# EUROPEAN PATENT APPLICATION

(11) **EP 4 091 659 A1**
(43) Date of publication of application: **23.11.2022**
(21) Application number: 20913655.5
(22) Date of filing: 02.12.2020
(51) Int. Cl.: A61M 25/09

(54) **GUIDE WIRE**

(30) Priority: 17.01.2020 JP 2020006042
(71) Applicant: Asahi Intecc Co., Ltd., Seto-shi, Aichi 489-0071 (JP)
(72) Inventor: KAWASHIMA, Naoya, Seto-shi, Aichi 489-0071 (JP)
(74) Representative: Prinz & Partner mbB
(86) International application number: PCT/JP2020/044834
(87) International publication number: WO 2021/145083

(57) **Abstract**

A guide wire includes a core shaft and a coil body wound around at least a portion of the core shaft on a proximal end side. The coil body is joined with the core shaft by a predetermined joint means at a position of a proximal end of the core shaft, and a first position along an axial direction of the guide wire between the proximal end and a distal end of the core shaft. The coil body is joined with the core shaft by welding at an axial specific position being at least one position within a range from the proximal end of the core shaft to the first position along the axial direction of the guide wire.

## Description

### TECHNICAL FIELD

The technique disclosed herein relates to a guide wire to be inserted into a blood vessel or the like.

### BACKGROUND

A method using a catheter is widely used for a method for treating or inspecting a constricted part or an occluded part (hereinafter referred to as a "lesion") in a blood vessel or the like. Typically, a guide wire is used to guide a catheter to a lesion in a blood vessel or the like. The guide wire includes a core shaft and a coil body wound around at least a portion of the core shaft on the proximal end side. The coil body is provided, for example, to make a proximal end side portion of the guide wire having an appropriate thickness without excessively increasing the rigidity of the proximal end side portion of the guide wire.

In a conventional guide wire, the coil body is joined to the core shaft by, for example, brazing at positions of a proximal end and a distal end of the guide wire (see, for example, Patent Literatures 1 and 2).

### CITATION LIST

### Patent Literature

Patent Literature 1: US 4003369B
Patent Literature 2: WO2010/018762A

### SUMMARY

### TECHNICAL PROBLEM

In a guide wire having a conventional configuration in which the coil body is joined to the core shaft at the positions of the proximal end and the distal end of the guide wire, the rotational force applied to the proximal end side portion of the guide wire is transmitted to the core shaft at only two locations, the proximal end and the distal end, and thus, there is room for improvement in terms of the torquability from the proximal end side to the distal end side. A configuration in which the entire coil body is continuously joined to the core shaft to improve the torquability of the guide wire is conceivable; however, in this configuration, the rigidity of the proximal end side portion of the guide wire is excessively high, and thus is not preferable. As described above, there is a problem with the conventional guide wire in that it is not possible to improve the torquability while avoiding excessively high rigidity of the proximal end side portion of the guide wire.

The present specification discloses a technique by which it is possible to solve the above-described problems.

### SOLUTION TO PROBLEM

The techniques disclosed herein can be realized in the following aspects, for example.

(1) A guide wire disclosed herein includes a core shaft and a coil body wound around at least a portion on a proximal end side of the core shaft. The coil body is joined with the core shaft by a predetermined joint means at a position of a proximal end of the core shaft, and a first position along an axial direction of the guide wire between the proximal end and a distal end of the core shaft. The coil body is joined with the core shaft by welding at an axial specific position being at least one position within a range along the axial direction of the guide wire from the proximal end of the core shaft to the first position. According to this guide wire, in addition to the coil body and the core shaft being joined at a position of the proximal end and the first position, the coil body and the core shaft are also joined by welding at an axial specific position being at least one position within a range from the proximal end to the first position, and thus, a rotational force applied to a proximal end side portion of the guide wire is transmitted to the core shaft at more locations, so that the torquability from the proximal end side to the distal end side is improved. In this guide wire, the coil body is not continuously joined to the core shaft as a whole, and the coil body and the core shaft are merely joined by welding at axial specific positions, and thus, the rigidity of the proximal end side portion of the guide wire is not excessively high. Therefore, according to this guide wire, it is possible to improve the torquability while avoiding an excessive increase in the rigidity of the proximal end side portion of the guide wire.

(2) The guide wire above may have a configuration in which the guide wire includes a plurality of the axial specific positions. According to such a configuration, the torquability of the guide wire can be effectively improved due to the presence of the welded locations at the plurality of axial specific positions.

(3) The guide wire above may have a configuration in which the plurality of axial specific positions are arranged at equal intervals along the axial direction of the guide wire. According to such a configuration, the welded locations of the coil body and the core shaft can be arranged in a well-balanced manner along the axial direction, and thus, the torquability of the guide wire can be further effectively improved.

(4) The guide wire above may have a configuration in which the coil body is joined with the core shaft by welding at a part along the circumferential direction of the guide wire at at least one of the plurality of axial specific positions. According to such a configuration, it is possible to limit the range affected by heat during welding, and it is possible to suppress changes in the characteristics of the guide wire due to welding.

(5) The guide wire above may have a configuration in which the coil body is joined with the core shaft by welding at a plurality of locations different from each other along the circumferential direction of the guide wire at at least one of the plurality of axial specific positions. According to such a configuration, the torquability of the guide wire can be effectively improved due to the presence of a plurality of welded locations at each of the plurality of axial specific positions.

(6) The guide wire above may have a configuration in which the plurality of locations different from each other along the circumferential direction of the guide wire are arranged at equal intervals along the circumferential direction of the guide wire. According to such a configuration, the welded locations between the coil body and the core shaft can be arranged in a well-balanced manner along the circumferential direction at each of the plurality of axial specific positions, and thus, the torquability of the guide wire can be further effectively improved.

(7) The guide wire above may have a configuration in which the number of welded locations at one of the plurality of axial specific positions is different from the number of welded locations at another of the plurality of the axial specific positions. According to such a configuration, a technician can recognize the rotation angle of the guide wire from the appearance of the guide wire, and thus, the operability and the convenience can be improved.

(8) The guide wire above may have a configuration in which a plurality of first combinations are arranged along the axial direction of the guide wire, the first combination being composed of a plurality of axial specific positions continuous along the axial direction of the guide wire in which a pattern of the number of welded locations at each of the plurality of axial specific positions is a specific pattern. According to such a configuration, the appearance of the guide wire can be made regular over the entire range from the proximal end of the core shaft to the first position, and a technician can easily recognize the rotation angle of the guide wire by the appearance of the guide wire, so that the operability and the convenience can be effectively improved.

(9) The guide wire above may have a configuration in which a position of welded locations along the circumferential direction of the guide wire at one of the plurality of axial specific positions differs from a position of welded locations along the circumferential direction of the guide wire at another of the plurality of the axial specific positions. According to such a configuration, the welded locations of the coil body and the core shaft can be arranged in a well-balanced manner along the circumferential direction, and the torquability of the guide wire can be further effectively improved.

(10) The guide wire above may have a configuration in which a plurality of second combinations are arranged along the axial direction of the guide wire, the second combination being composed of a plurality of axial specific positions continuous along the axial direction of the guide wire in which a pattern of a position of a welded location along the circumferential direction of the guide wire at each of the plurality of axial specific positions is a specific pattern. According to such a configuration, the welded locations between the coil body and the core shaft can be arranged in a well-balanced manner along the circumferential direction over the entire range from the proximal end of the core shaft to the first position, and thus, the torquability of the guide wire can be further effectively improved. In addition, according to such a configuration, it is not necessary to stop the axial movement of the work during welding, and thus, the time required for welding can be shortened and efficient manufacturing of the guide wire can be achieved.

(11) The guide wire above may have a configuration in which for the plurality of axial specific positions included in the second combination, amounts of deviation of positions of welded locations along the circumferential direction of the guide wire between any two of the plurality of axial specific positions are equal to each other, the two axial specific positions being adjacent to each other along the axial direction of the guide wire. According to such a configuration, the welded locations of the coil body and the core shaft can be arranged in an extremely well-balanced manner along the circumferential direction over the entire range from the proximal end of the core shaft to the first position, and thus, the torquability of the guide wire can be improved extremely effectively.

(12) The guide wire above may have a configuration in which the coil body is joined by welding with the core shaft over the entire circumference along the circumferential direction of the guide wire at at least one of the axial specific positions. According to such a configuration, the welding strength per location can be increased, and thus, the torquability of the guide wire can be effectively improved. In addition, according to such a configuration, a technician can easily recognize the insertion length of the guide wire from the appearance and/or the tactile sensation of the guide wire, and thus, the operability and the convenience can be improved.

(13) The guide wire above may have a configuration in which a position of a welded location along the circumferential direction of the guide wire at each of the plurality of axial specific positions is identical to each other. According to such a configuration, every time the guide wire is rotated by 360° in the circumferential direction, intentional bouncing can be generated, and a technician can easily perform a specific operation of the guide wire (for example, escape from a false cavity or guide to a branch pipe), so that the operability and the convenience can be improved. In addition, according to such a configuration, it is not necessary to stop the axial movement of the work during welding, and thus, the time required for welding can be shortened and efficient manufacturing of the guide wire can be achieved.

(14) The guide wire above may have a configuration in which the predetermined joint means is brazing. According to such a configuration, melting of the base material can be suppressed by adopting joining by brazing at the position of the proximal end of the core shaft and the above-mentioned first position, and by adopting joining by welding at each of the plurality of axial specific positions, high-strength joint can be achieved.

The techniques disclosed herein can be achieved in various forms, for example, in the form of a guide wire, a method for manufacturing the guide wire, and the like.

### BRIEF DESCRIPTION OF DRAWINGS

FIG. 1 is an explanatory diagram schematically illustrating a configuration of a guide wire 100 in a first embodiment.
FIG. 2 is an explanatory diagram schematically illustrating a configuration of the guide wire 100 in the first embodiment.
FIG. 3 is an explanatory diagram illustrating an arrangement of welded portions 50 in the guide wire 100 of the first embodiment.
FIG. 4 is an explanatory diagram illustrating an arrangement of the welded portions 50 in the guide wire 100 of the first embodiment.
FIG. 5 is an explanatory diagram illustrating in detail the external shape of the guide wire 100 of the first embodiment.
FIG. 6 is an explanatory diagram illustrating an arrangement of the welded portions 50 in a guide wire 100a of a second embodiment.
FIG. 7 is an explanatory diagram illustrating an arrangement of the welded portions 50 in the guide wire 100a of the second embodiment.
FIG. 8 is an explanatory diagram illustrating in detail the shape of the guide wire 100a of the second embodiment.
FIG. 9 is an explanatory diagram illustrating an arrangement of the welded portions 50 in a guide wire 100b of a third embodiment.
FIG. 10 is an explanatory diagram illustrating an arrangement of the welded portions 50 in the guide wire 100b of the third embodiment.
FIG. 11 is an explanatory diagram illustrating in detail the shape of the guide wire 100b of the third embodiment.
FIG. 12 is an explanatory diagram illustrating an arrangement of the welded portions 50 in a guide wire 100c of a fourth embodiment.
FIG. 13 is an explanatory diagram illustrating an arrangement of the welded portions 50 in the guide wire 100c of the fourth embodiment.
FIG. 14 is an explanatory diagram illustrating an arrangement of the welded portions 50 in a guide wire 100d of a fifth embodiment.
FIG. 15 is an explanatory diagram illustrating an arrangement of the welded portions 50 in the guide wire 100d of the fifth embodiment.
FIG. 16 is an explanatory diagram illustrating an arrangement of the welded portions 50 in a guide wire 100e of a sixth embodiment.
FIG. 17 is an explanatory diagram illustrating an arrangement of the welded portions 50 in the guide wire 100e of the sixth embodiment.

### EMBODIMENTS

### A. First Embodiment:

### A-1. Basic Configuration of Guide Wire 100:

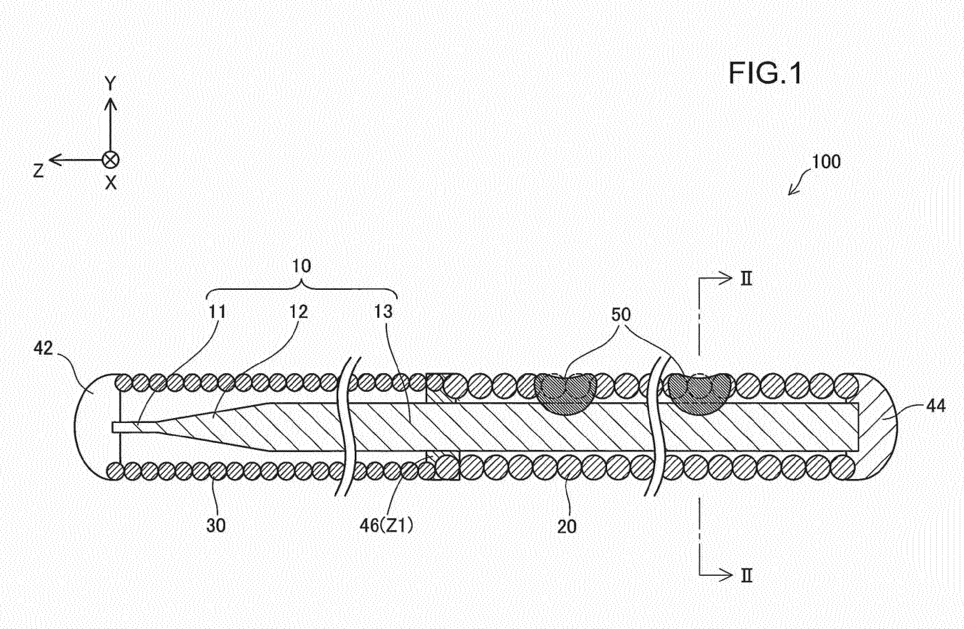

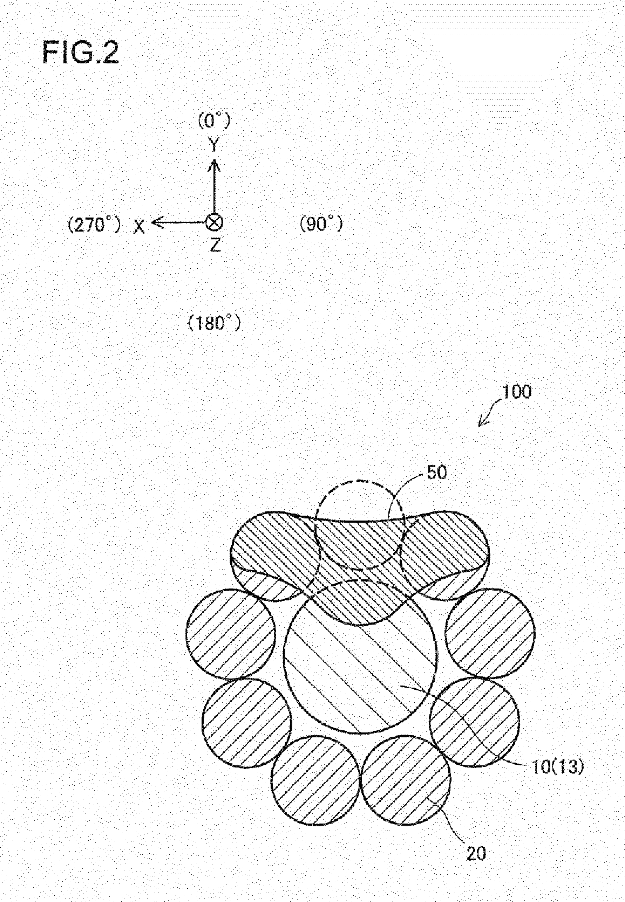

FIG. 1 and FIG. 2 are explanatory diagrams schematically illustrating a configuration of a guide wire 100 in a first embodiment. FIG. 1 illustrates the configuration of a longitudinal cross section (YZ cross section) of the guide wire 100, and FIG. 2 illustrates the configuration of a transverse cross section (XY cross section) of the guide wire 100 at the position II-II in FIG. 1. In FIG. 1, a part of the guide wire 100 is not illustrated. In FIG. 1, a Z-axis positive direction side is a distal end side (far side) to be inserted into a body, and a Z-axis negative direction side is a proximal end side (near side) operated by a technician such as a doctor. FIG. 1 illustrates a state where the guide wire 100 has an overall linear shape substantially parallel to the Z-axis direction, however, the guide wire 100 has enough flexibility to be bent.

In this specification, for convenience of explanation, the guide wire 100 is presumed to be in the state illustrated in FIG. 1, and the Z-axis direction is referred to as the "axial direction of the guide wire 100" or simply the "axial direction", and the direction of rotation about the Z axis is referred to as the "circumferential direction of the guide wire 100" or simply the "circumferential direction". As illustrated in FIG. 2, regarding the circumferential direction, the positive direction of the Y axis is referred to as an upward direction or a 0° direction, the negative direction of the Y axis is referred to as a downward direction or a 180° direction, and the negative direction of the X axis is referred to as a near side direction or a 90° direction, and the positive direction of the X-axis is referred to as a far side direction or a 270° direction.

The guide wire 100 is a long medical device to be inserted into a blood vessel or the like to guide a catheter to a lesion (a constricted part or an occluded part) in a blood vessel or the like. The total length of the guide wire 100 is, for example, about 1500 mm to 2000 mm, and the outer diameter of the guide wire 100 is, for example, about 0.5 to 1.2 mm.

The guide wire 100 includes a core shaft 10, a proximal end side coil body 20, a distal end side coil body 30, a distal end side joint part 42, a proximal end side joint part 44, and an intermediate joint part 46.

The core shaft 10 is a long member having a smaller diameter on the distal end side and a larger diameter on the proximal end side. More specifically, the core shaft 10 includes a small-diameter portion 11 having a rod shape, a large-diameter portion 13 being located on the proximal end side with respect to the small-diameter portion 11 and having a rod shape having a larger diameter than the small-diameter portion 11, and a tapered portion 12 located between the small-diameter portion 11 and the large-diameter portion 13 and whose diameter gradually increases going from a boundary location with the small-diameter portion 11 toward a boundary location with the large-diameter portion 13. A shape of the transverse cross section (XY cross section) at each locations of the core shaft 10 can take any shape, and is, for example, a circular shape or a flat plate shape. The outer diameter of the large-diameter portion 13 is, for example, about 0.2 to 0.6 mm.

A known material is used as the material of the core shaft 10; for example, a metal material, and more specifically, stainless steel (SUS302, SUS304, SUS316, and the like), superelastic alloy such as Ni-Ti alloy, piano wire, nickel-chromium alloy, cobalt alloy, tungsten and the like is used. The core shaft 10 may be formed of the same material as a whole, or may be configured of different materials for each part.

The proximal end side coil body 20 is a coil-shaped member formed into a hollow cylindrical shape by winding a wire into a spiral shape. The proximal end side coil body 20 is wound around a part on the proximal end side of the core shaft 10 (for example, a portion having a distance of 700 mm to 1200 mm or less from the proximal end). The portion of the guide wire 100 around which the proximal end side coil body 20 is wound is a portion that is mainly gripped by a technician and not inserted into a body. In the present embodiment, the proximal end side coil body 20 has a configuration in which one wire is tightly wound. The distal end side coil body 30 is a coil-shaped member formed into a hollow cylindrical shape by winding a wire into a spiral shape. The distal end side coil body 30 is wound around a portion of the core shaft 10 on the distal end side (for example, a remaining portion where the proximal end side coil body 20 is not wound). The portion of the guide wire 100 around which the distal end side coil body 30 is wound is mainly a portion to be inserted into a body. In the present embodiment, the distal end side coil body 30 has a configuration in which one wire is tightly wound. In the present embodiment, an outer diameter dimension of the proximal end side coil body 20 and an outer diameter dimension of the distal end side coil body 30 are substantially the same. The diameter of the wire of the proximal end side coil body 20 is larger than the diameter of the wire of the distal end side coil body 30, and is, for example, about 0.1 to 0.3 mm. The proximal end side coil body 20 is an example of a coil body in the claims.

Known materials are used as the material of the proximal end side coil body 20 and the distal end side coil body 30, and for example, a metal material, more specifically, stainless steel (SUS302, SUS304, SUS316, and the like), superelastic alloy such as Ni-Ti alloy, piano wire, nickel-chromium alloy, cobalt alloy, tungsten and the like are used. The proximal end side coil body 20 and the distal end side coil body 30 may be configured of the same material or may be configured of different materials. The proximal end side coil body 20 and the distal end side coil body 30 may be configured of the same material as a whole, or may be configured of different materials for each part.

The distal end side joint part 42 is a member that joins the distal end of the core shaft 10 and the distal end of the distal end side coil body 30. That is, the distal end of the core shaft 10 and the distal end of the distal end side coil body 30 are embedded in the distal end side joint part 42 for fixation. The outer circumferential surface on the distal end side of the distal end side joint part 42 is a smooth surface (for example, a substantially hemispherical surface). The proximal end side joint part 44 is a member that joins the proximal end of the core shaft 10 and the proximal end of the proximal end side coil body 20. That is, the proximal end of the core shaft 10 and the proximal end of the proximal end side coil body 20 are embedded in the proximal end side joint part 44 for fixation. The intermediate joint part 46 is located at a predetermined position along the axial direction between the proximal end and the distal end of the core shaft 10 (hereinafter, referred to as "intermediate position Z1"), and is a member that joins the core shaft 10, the proximal end of the distal end side coil body 30, and the distal end of the proximal end side coil body 20. That is, the proximal end of the distal end side coil body 30 and the distal end of the proximal end side coil body 20 are embedded in the inside of the intermediate joint part 46 formed on the outer circumferential surface of the core shaft 10 at the intermediate position Z1 for fixation. The intermediate position Z 1 is an example of a first position in the claims.

Known materials are used as materials of the distal end side joint part 42, the proximal end side joint part 44, and the intermediate joint part 46, and for example, a brazing material (aluminum alloy brazing, silver brazing, gold brazing, and the like), metal solder (Ag-Sn alloy, Au-Sn alloy, and the like), adhesive (epoxy-based adhesive and the like) and the like are used. In the present embodiment, a brazing material is used as a material of the distal end side joint part 42, the proximal end side joint part 44, and the intermediate joint part 46. That is, in the present embodiment, the proximal end side coil body 20 is joined to the core shaft 10 by brazing at the position of the proximal end of the core shaft 10 and the intermediate position Z1. Brazing is an example of a predetermined joint means in the claims.

A part or all of the guide wire 100 may be coated with a known coating agent.

### A-2. Detailed Configuration for Bonding Core Shaft 10 and Proximal End

### Side Coil Body 20:

Next, a detailed configuration of joining between the core shaft 10 and the proximal end side coil body 20 in the guide wire 100 of the present embodiment will be described. As illustrated in FIGS. 1 and 2, in the guide wire 100 of the present embodiment, the proximal end side coil body 20 is joined to the core shaft 10 by welding. In other words, the guide wire 100 of the present embodiment has welded portions 50 joining the proximal end side coil body 20 and the core shaft 10. The welded portions 50 are portions formed by heating and melting the core shaft 10 and the proximal end side coil body 20 as a base material, and then solidifying them. In FIGS. 1 and 2, for reference, a state of the base material before the welded portions 50 are formed is indicated by dashed lines in the vicinity of the welded portions 50. The outer diameter of the welded portions 50 is, for example, about 70% to 90% of the outer diameter of the guide wire 100. The penetration depth of the welded portions 50 is, for example, about 25% to 50% of the outer diameter of the guide wire 100. Any known welding means can be adopted as the welding means between the proximal end side coil body 20 and the core shaft 10; however, using laser welding, which can obtain a deep penetration depth with a small amount of heat input, is preferable.

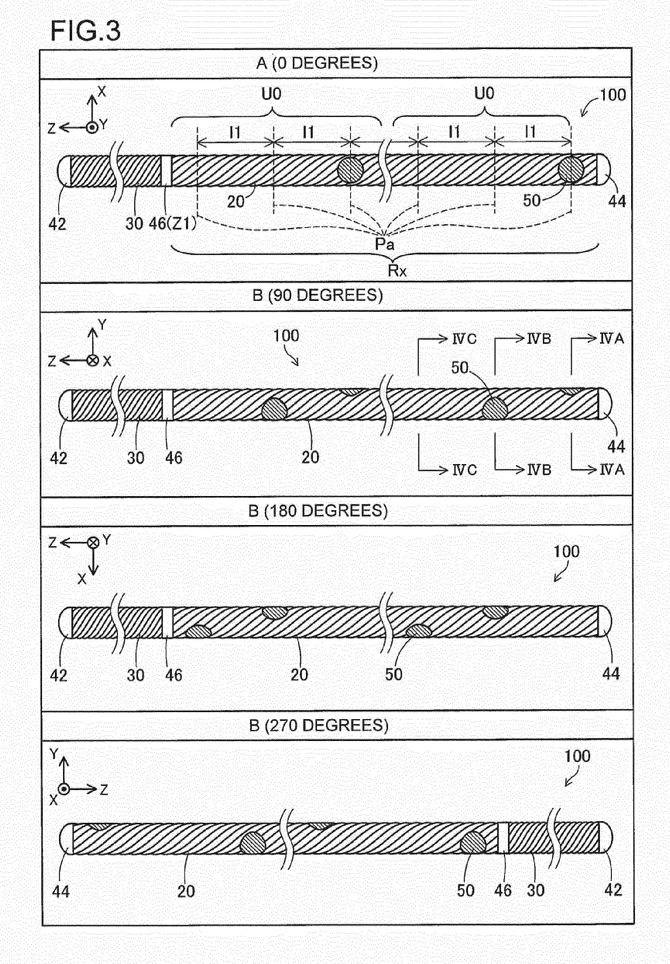

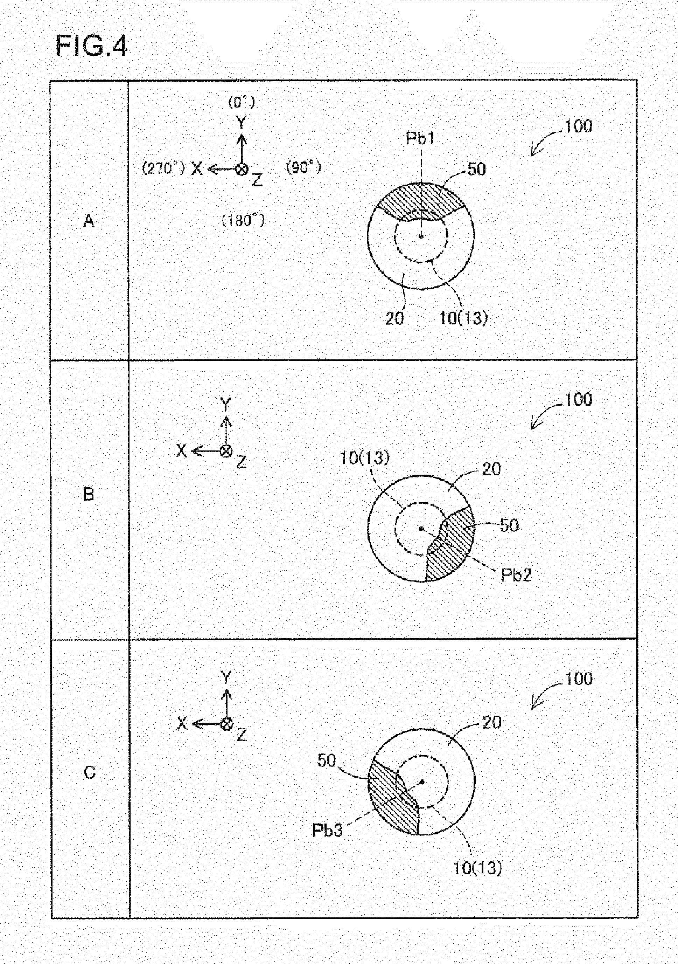

FIGS. 3 and 4 are explanatory diagrams illustrating the arrangement of the welded portions 50 in the guide wire 100 of the first embodiment. Column A in FIG. 3 schematically illustrates an external configuration of the guide wire 100 as viewed from above (0° direction), column B in FIG. 3 schematically illustrates an external configuration of the guide wire 100 as viewed from the near side (90° direction), column C in FIG. 3 schematically illustrates an external configuration of the guide wire 100 as viewed from below (180° direction), and column D in FIG. 3 schematically illustrates an external configuration of the guide wire 100 as viewed from the far side (270° direction). Column A in FIG. 4 schematically illustrates a configuration of a transverse cross section (XY cross section) of the guide wire 100 at a position IVA-IVA in column B in FIG. 3, column B in FIG. 4 schematically illustrates a configuration of a transverse cross section (XY cross section) of the guide wire 100 at a position IVB-IVB in column B in FIG. 3, and column C in FIG. 4 schematically illustrates a configuration of a transverse cross section (XY cross section) of the guide wire 100 at a position IVC-IVC in column B in FIG. 3. In columns A to C in FIG. 4, details of the configuration of the guide wire 100 other than the welded portions 50 are omitted.

As illustrated in FIGS. 3 and 4, in the guide wire 100 of the first embodiment, in a range along the axial direction from the proximal end of the core shaft 10 (that is, the position of the proximal end side joint part 44) to the intermediate position Z 1 (that is, the position of the intermediate joint part 46) (hereinafter, referred to as "specific range Rx"), a plurality of axial specific positions Pa are set, and one welded portion 50 is formed at each axial specific position Pa. In the guide wire 100 of the present embodiment, the proximal end side coil body 20 is joined to the core shaft 10 by welding in a part along the circumferential direction. That is, as illustrated in FIG. 4, each welded portion 50 is not formed to cover the entire outer periphery of the guide wire 100 in the XY cross section, but is formed to cover a part of the outer periphery. In the guide wire 100 of the present embodiment, the plurality of axial specific positions Pa are arranged at equal intervals along the axial direction. That is, distances 11 between two axial specific positions Pa adjacent to each other along the axial direction are all the same, for example, about 10 mm to 50 mm. In this description, "equal to (or the same as)" includes "almost equal to (or almost the same as)", and here, "mostly equal to (or mostly the same as)" means that the difference between two values to be compared is 10% or less of the larger value.

For example, if the length of the proximal end side coil body 20 is 950 mm, the distance 11 along the axial direction between two axial specific positions Pa adjacent to each other is 30 mm, and the distance from the proximal end of the guide wire 100 to the nearest welded portion 50 is 10 mm, the axial specific positions Pa are arranged at 32 locations within the specific range Rx of the guide wire 100.

In the guide wire 100 of the first embodiment, the position of the welded portion 50 (welded location) along the circumferential direction at a certain axial specific position Pa (hereinafter referred to as "circumferential position Pb of the welded portion 50") is different from a circumferential position Pb of the welded portion 50 at another axial specific position Pa. More specifically, a circumferential position Pb1 of the welded portion 50 at the axial specific position Pa whose cross section is illustrated in column A in FIG. 4 is a 0° position, a circumferential position Pb2 of the welded portion 50 at the axial specific position Pa whose cross section is illustrated in column B in FIG. 4 is a 120° position, and a circumferential position Pb3 of the welded portion 50 at the axial specific position Pa whose cross section is illustrated in column C in FIG. 4 is a 240° position. The circumferential position Pb of the welded portion 50 means the position of the center of the outer circumferential surface of the welded portion 50 in the cross section (XY cross section) orthogonal to the axial direction. As described above, in the guide wire 100 of the first embodiment, the circumferential positions Pb of the welded portions 50 at the three axial specific positions Pa arranged in the axial direction are deviated by 120° from each other.

As illustrated in FIG. 3, in the guide wire 100 of the first embodiment, a combination of three axial specific positions Pa continuous in the axial direction (hereinafter referred to as "unit combination U0") in which the pattern of the circumferential positions Pb of the welded portions 50 at the axial specific positions Pa is the above-mentioned specific pattern (0° position, 120° position, and 240° position) is repeatedly arranged along the axial direction. That is, a regular pattern is formed in which the circumferential positions Pb of the welded portions 50 at the axial specific positions Pa are arranged in the order of 0° position, 120° position, 240° position, 0° position, 120° position, 240° position, and so on, starting from a position near the proximal end of the guide wire 100. The unit combination U0 in the present embodiment is an example of a second combination in the claims.

A method of forming each welded portion 50 in the guide wire 100 of the first embodiment is, for example, as described below. That is, a work having a configuration in which the proximal end side coil body 20 and the core shaft 10 are joined by the proximal end side joint part 44 and the intermediate joint part 46 is manufactured. The work is moved in the axial direction at a constant speed while being rotated in the circumferential direction at a constant speed, and the work is irradiated with laser light from a laser welder installed at a predetermined position at regular time intervals. By irradiating the work with laser light, the core shaft 10 and the proximal end side coil body 20 as the base material are heated and melted, and then solidified to form the welded portion 50. Through the above steps, the welded portion 50 is formed at each axial specific position Pa. If the outer diameter, the peak power, and the pulse width of the laser light to be irradiated are adjusted in forming the welded portions 50, the outer diameter and the penetration depth of the welded portions 50 to be formed can be adjusted.

### A-3. Effects of First Embodiment:

As described above, the guide wire 100 of the first embodiment includes the core shaft 10 and the proximal end side coil body 20 wound around a part on the proximal end side of the core shaft 10. At the position of the proximal end of the core shaft 10 and the intermediate position Z1 between the proximal end and the distal end of the core shaft 10 along the axial direction, the proximal end side coil body 20 is joined to the core shaft 10. At the axial specific position Pa being at least one position within the specific range Rx from the proximal end of the core shaft 10 to the intermediate position Z1 along the axial direction, the proximal end side coil body 20 is joined to the core shaft 10 by welding (the welded portions 50 for joining the proximal end side coil body 20 and the core shaft 10 are formed).

Thus, in the guide wire 100 of the first embodiment, the proximal end side coil body 20 and the core shaft 10, in addition to the proximal end side joint part 44 and the intermediate joint part 46, are joined by the welded portions 50 formed at the axial specific positions Pa, and thus, compared with a configuration without such welded portions 50, a rotational force applied to the proximal end side portion of the guide wire 100 is transmitted to the core shaft 10 at more locations, improving the torquability from the proximal end side to the distal end side. In the guide wire 100 of the first embodiment, the proximal end side coil body 20 is not continuously joined as a whole to the core shaft 10, and the proximal end side coil body 20 and the core shaft 10 are merely joined by welding at the axial specific positions Pa, and thus, rigidity of the proximal end side portion of the guide wire 100 is not excessively high. Therefore, the guide wire 100 of the first embodiment can improve the torquability while avoiding an excessive increase in the rigidity of the proximal end side portion of the guide wire 100.

In the guide wire 100 of the first embodiment, there are the plurality of axial specific positions Pa within the specific range Rx. In other words, a plurality of the welded portions 50 are formed within the specific range Rx of the guide wire 100. Therefore, according to the guide wire 100 of the first embodiment, the torquability can be effectively improved due to the presence of the plurality of welded portions 50.

In the guide wire 100 of the first embodiment, the plurality of axial specific positions Pa are arranged at equal intervals along the axial direction. In other words, the plurality of welded portions 50 are formed at the axial specific positions Pa arranged at equal intervals along the axial direction. Therefore, according to the guide wire 100 of the first embodiment, the welded locations of the proximal end side coil body 20 and the core shaft 10 can be arranged in a well-balanced manner along the axial direction, and thus, the torquability can be further effectively improved.

In the guide wire 100 of the first embodiment, the proximal end side coil body 20 is joined to the core shaft 10 by welding in a part along the circumferential direction at each axial specific position Pa. Therefore, according to the guide wire 100 of the first embodiment, a range affected by heat at the time of welding can be limited compared with a configuration in which the proximal end side coil body 20 is joined to the core shaft 10 by welding over the entire circumference in the circumferential direction, making it possible to suppress changes in the characteristics of the guide wire 100 due to welding.

In the guide wire 100 of the first embodiment, the circumferential position Pb of the welded portion 50 at one axial specific position Pa is different from the circumferential position Pb of the welded portion 50 at another axial specific position Pa. Therefore, according to the guide wire 100 of the first embodiment, the welded locations of the proximal end side coil body 20 and the core shaft 10 can be arranged in a well-balanced manner along the circumferential direction, and thus, the torquability can be further effectively improved.

In the guide wire 100 of the first embodiment, the unit combination U0 is a combination of the plurality of axial specific positions Pa continuous along the axial direction, and a plurality of the unit combinations U0 having the pattern of the circumferential positions Pb of the welded portions 50 at the axial specific positions Pa being a specific pattern are arranged along the axial direction. Therefore, according to the guide wire 100 of the first embodiment, the welded locations of the proximal end side coil body 20 and the core shaft 10 can be arranged in a well-balanced manner along the circumferential direction over the entire specific range Rx of the guide wire 100, making it possible to further effectively improve the torquability. Moreover, according to the guide wire 100 of the first embodiment, it is not necessary to stop the axial movement of the work at the time of laser welding for forming the welded portions 50, and thus, the time required for forming the welded portions 50 can be shortened, making it possible to improve the efficiency of manufacturing the guide wire 100.

In the guide wire 100 of the first embodiment, for the plurality of axial specific positions Pa included in the unit combination U0, amounts of deviation of the circumferential positions Pb of the welded portions 50 at any two axial specific positions Pa adjacent to each other along the axial direction are equal to each other (that is, 120°). Therefore, according to the guide wire 100 of the first embodiment, the welded locations of the proximal end side coil body 20 and the core shaft 10 can be arranged in an extremely well-balanced manner along the circumferential direction over the entire specific range Rx of the guide wire 100, making it possible to improve the torquability extremely effectively.

In the guide wire 100 of the first embodiment, the means of joining between the proximal end side coil body 20 and the core shaft 10 at the position of the proximal end of the core shaft 10 and the intermediate position Z1 is brazing. Therefore, according to the guide wire 100 of the first embodiment, melting of the base material can be suppressed by adopting joining by brazing at the position of the proximal end of the core shaft 10 and the intermediate position Z1, and high-strength joint can be achieved by adopting joining by welding at the axial specific positions Pa.

### A-4. Details of External Shape of Guide Wire 100:

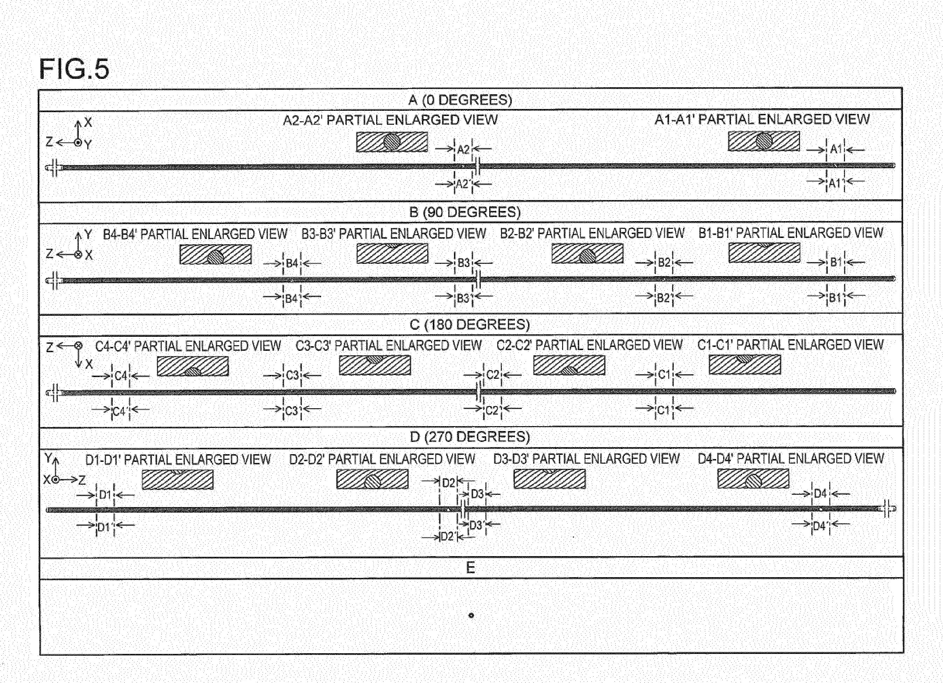

The external shape of the guide wire 100 will be described in detail. FIG. 5 is an explanatory diagram illustrating in detail the external shape of the guide wire 100 of the first embodiment. Column A in FIG. 5 schematically illustrates a top view of the guide wire 100 (as viewed from the 0° direction), column B in FIG. 5 schematically illustrates a front view of the guide wire 100 (as viewed from the 90° direction), column C in FIG. 5 schematically illustrates a bottom view of the guide wire 100 (as viewed from the 180° direction), column D in FIG. 5 schematically illustrates a rear view of the guide wire 100 (as viewed from the 270° direction), and column E in FIG. 5 schematically illustrates a left side view of the guide wire 100 (as viewed from the Z-axis positive direction). A right side view of the guide wire 100 (as viewed from the Z-axis negative direction) is the same as the left side view of the guide wire 100 illustrated in column E in FIG. 5, and is therefore omitted.

In columns A to C in FIG. 5, of the two omitted parts, the dimension on the drawing of the omitted part on the right side is 125.8 cm, the dimension on the drawing of the omitted part on the left side is 133.1 cm, and the dimension on the drawing in the entire length direction (Z-axis direction) of the guide wire 100 is 282.6 cm. In column D in FIG. 5, of the two omitted parts, the dimension on the drawing of the omitted part on the left side is 125.8 cm, the dimension on the drawing of the omitted part on the right side is 133.1 cm, and the dimension on the drawing in the entire length direction (Z-axis direction) of the guide wire 100 is 282.6 cm. In columns A to E in FIG. 5, the portion represented by the solid line is the proximal end side coil body 20 of the guide wire 100, and is a portion for which design registration is to be received as a partial design. In columns A to D in FIG. 5, a partially enlarged view of each part is also illustrated.

### B. Second Embodiment:

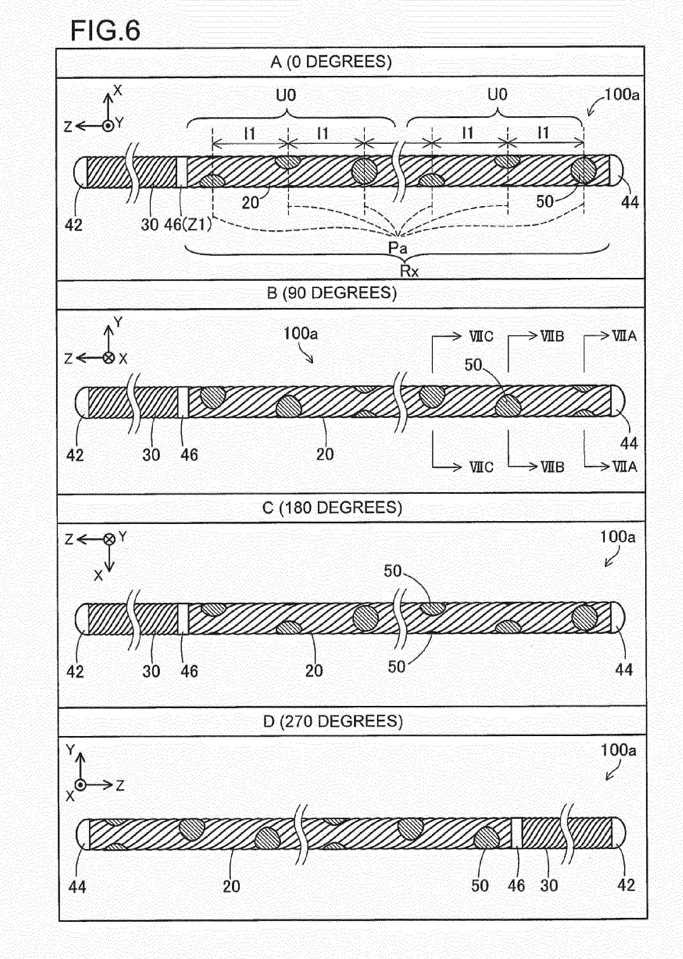

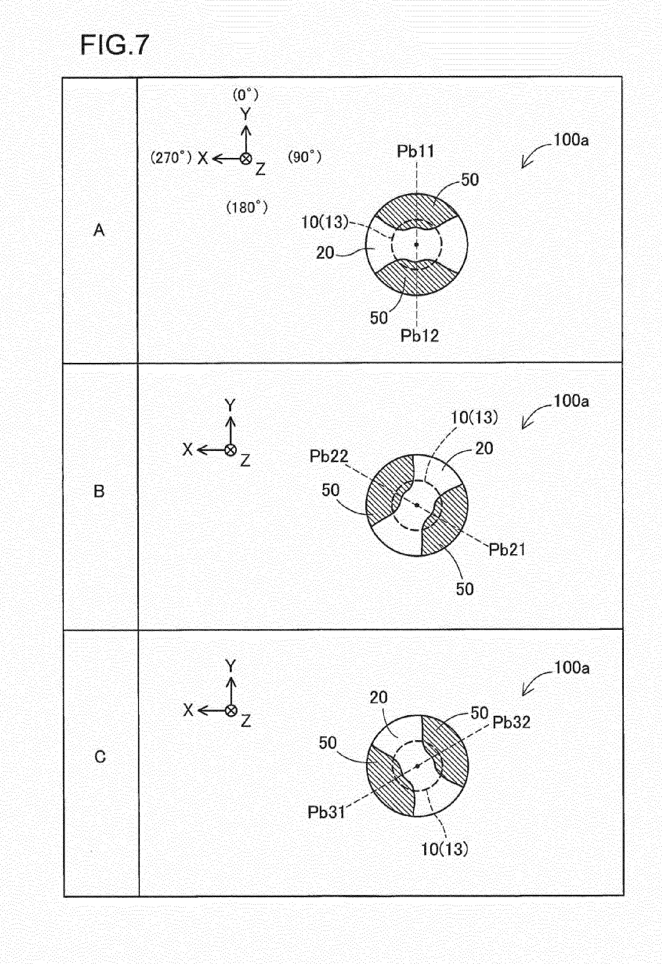

FIGS. 6 and 7 are explanatory diagrams illustrating the arrangement of the welded portions 50 in a guide wire 100a of a second embodiment. In the following, a configuration the same as that of the guide wire 100 of the first embodiment described above out of the configuration of the guide wire 100a of the second embodiment will be referred to by the same reference numerals, and a description thereof will be omitted where appropriate. Column A in FIG. 6 schematically illustrates an external configuration of the guide wire 100a as viewed from above (0° direction), column B in FIG. 6 schematically illustrates an external configuration of the guide wire 100a as viewed from the near side (90° direction), column C in FIG. 6 schematically illustrates an external configuration of the guide wire 100a as viewed from below (180° direction), and column D in FIG. 6 schematically illustrates an external configuration of the guide wire 100a as viewed from the far side (270° direction). Column A in FIG. 7 schematically illustrates a configuration of a transverse cross section (XY cross section) of the guide wire 100a at a position VIIA-VIIA in column B in FIG. 6, column B in FIG. 7 schematically illustrates a configuration of a transverse cross section (XY cross section) of the guide wire 100a at a position VIIB-VIIB in column B in FIG. 6, and column C in FIG. 7 schematically illustrates a configuration of a transverse cross section (XY cross section) of the guide wire 100a at a position VIIC-VIIC in column B in FIG. 6. In columns A to C in FIG. 7, the details of the configuration of the guide wire 100a other than the welded portion 50 are omitted.

The guide wire 100a of the second embodiment is different from the guide wire 100 of the first embodiment in the arrangement of the welded portions 50. That is, as illustrated in FIGS. 6 and 7, in the guide wire 100a of the second embodiment, similar to the guide wire 100 of the first embodiment, the plurality of axial specific positions Pa arranged at equal intervals along the axial direction are set within the specific range Rx. However, in the guide wire 100a of the second embodiment, two welded portions 50 are formed at each axial specific position Pa. As illustrated in FIG. 7, the positions where the two welded portions 50 are formed at each axial specific position Pa are arranged at equal intervals (distances shifted by 180°) along the circumferential direction.

In the guide wire 100a of the second embodiment, the circumferential positions Pb of the welded portions 50 at one axial specific position Pa are different from the circumferential positions Pb of the welded portions 50 at another axial specific positions Pa. More specifically, the circumferential positions Pb11 and Pb12 of the two welded portions 50 at the axial specific position Pa whose cross section is illustrated in column A in FIG. 7 are the 0° position and the 180° position, respectively; the circumferential positions Pb21 and Pb22 of the two welded portions 50 at the axial specific position Pa whose cross section is illustrated in column B in FIG. 7 are the 120° position and the 300° position, respectively; and the circumferential positions Pb31 and Pb32 of the two welded portions 50 at the axial specific position Pa whose cross section is illustrated in column C in FIG. 7 are the 240° position and the 60° position, respectively. As described above, in the guide wire 100a of the second embodiment, the circumferential positions Pb (Pb11, Pb21, Pb31) of one of the welded portions 50 at each of the three axial specific positions Pa arranged in the axial direction are displaced by 120° from each other. The circumferential positions Pb (Pb12, Pb22, Pb32) of the other welded portion 50 are also displaced by 120° from each other.

As illustrated in FIG. 6, in the guide wire 100a of the second embodiment, the patterns of the circumferential positions Pb of one welded portion 50 and the other welded portion 50 at each axial specific position Pa are the above-mentioned specific patterns (a pattern of the 0° position, 120° position, and 240° position, and a pattern of the 180° position, 300° position, and 60° position), and a combination of three axial specific positions Pa continuous in the axial direction (unit combination U0) is repeatedly arranged along the axial direction. That is, focusing on the one welded portion 50 at each axial specific position Pa, the pattern is a regular pattern in which the circumferential position Pb of the welded portion 50 at each axial specific position Pa is in the order of 0° position, 120° position, 240° position, 0° position, 120° position, 240° position, and so on, starting from a position near the proximal end of the guide wire 100a. Similarly, focusing on the other welded portion 50 at each axial specific position Pa, the pattern is a regular pattern in which the circumferential position Pb of the welded portion 50 at each axial specific position Pa is in the order of 180° position, 300° position, 60° position, 180° position, 300° position, 60° position, and so on, starting from a position near the proximal end of the guide wire 100a. The unit combination U0 in the present embodiment is an example of a second combination in the claims.

The method of forming the welded portions 50 in the guide wire 100a of the second embodiment is, for example, as described below. That is, a work having a configuration in which the proximal end side coil body 20 and the core shaft 10 are joined by the proximal end side joint part 44 and the intermediate joint part 46 is manufactured. The work is moved in the axial direction at a constant speed while being rotated in the circumferential direction at a constant speed, and the work is irradiated with laser light from a laser welder installed at a predetermined position at regular time intervals. By irradiating the work with laser light, the core shaft 10 and the proximal end side coil body 20 as the base material are heated and melted, and then solidified to form the welded portion 50. Through the above steps, one welded portion 50 is formed at each axial specific position Pa. After that, the work is moved in the direction opposite to the axial direction at a constant speed while rotating in the circumferential direction at a constant speed, and the work is irradiated with laser light from a laser welder installed at a predetermined position at regular time intervals. By irradiating the work with laser light, the core shaft 10 and the proximal end side coil body 20 as the base material are heated and melted, and then solidified to form the welded portion 50. Through the above steps, the other welded portion 50 at each axial specific position Pa is formed, and as a result, two welded portions 50 are formed at each axial specific position Pa.

As described above, in the guide wire 100a of the second embodiment, similar to the guide wire 100 of the first embodiment, the proximal end side coil body 20 and the core shaft 10 are joined by welding at the axial specific position Pa being at least one position within the specific range Rx (the welded portions 50 for joining the proximal end side coil body 20 and the core shaft 10 are formed). Therefore, according to the guide wire 100a of the second embodiment, as in the guide wire 100 of the first embodiment, the torquability can be improved while avoiding an excessive increase in the rigidity of the proximal end side portion of the guide wire 100a.

In the guide wire 100a of the second embodiment, similar to the guide wire 100 of the first embodiment, the plurality of axial specific positions Pa exist within the specific range Rx, and thus, the torquability can be effectively improved due to the presence of the plurality of welded portions 50. In the guide wire 100a of the second embodiment, similar to the guide wire 100 of the first embodiment, the plurality of axial specific positions Pa are arranged at equal intervals along the axial direction of the core shaft 10, and thus, the welded locations of the proximal end side coil body 20 and the core shaft 10 can be arranged in a well-balanced manner along the axial direction, so that the torquability can be further effectively improved.

In the guide wire 100a of the second embodiment, at each axial specific position Pa, the proximal end side coil body 20 is joined by welding with the core shaft 10 at a plurality of different positions along the circumferential direction. Therefore, according to the guide wire 100a of the second embodiment, the torquability can be effectively improved due to the presence of the plurality of welded portions 50 at each axial specific position Pa. In the guide wire 100a of the second embodiment, the circumferential positions Pb of the welded portions 50 at each axial specific position Pa are arranged at equal intervals along the circumferential direction. Therefore, according to the guide wire 100a of the second embodiment, the welded locations of the proximal end side coil body 20 and the core shaft 10 can be arranged in a well-balanced manner along the circumferential direction at each axial specific position Pa, and thus, the torquability can be further effectively improved.

As will be described below, the guide wire 100a of the second embodiment has the configuration similar to that of the guide wire 100 of the first embodiment, and therefore exhibits the effect similar to that of the guide wire 100 of the first embodiment. That is, in the guide wire 100a of the second embodiment, the circumferential position Pb of the welded portion 50 at one axial specific position Pa is different from the circumferential position Pb of the welded portion 50 at another axial specific position Pa, and thus, the welded locations of the proximal end side coil body 20 and the core shaft 10 can be arranged in a well-balanced manner along the circumferential direction, so that the torquability can be further effectively improved. In the guide wire 100a of the second embodiment, the unit combination U0 is a combination of the plurality of axial specific positions Pa continuous along the axial direction, and a plurality of the unit combinations U0 in which the pattern of the circumferential positions Pb of the welded portions 50 at each axial specific position Pa is a specific pattern are arranged along the axial direction, and thus, the welded locations of the proximal end side coil body 20 and the core shaft 10 can be arranged in a well-balanced manner along the circumferential direction over the entire specific range Rx in the guide wire 100a, so that it is possible to further effectively improve the torquability. It is also not necessary to stop the axial movement of the work during laser welding to form the welded portions 50 for each of the two welded portions 50 at each axial specific position Pa, and thus, the time required for forming the welded portions 50 can be shortened, so that efficient manufacturing of the guide wire 100a can be achieved. In the guide wire 100a of the second embodiment, with respect to the plurality of axial specific positions Pa included in the unit combination U0, the amount of deviation of the positions of the one welded portion 50 and the other welded portion 50 at any two axial specific positions Pa adjacent to each other along the axial direction is the same (that is, 120°), and thus, the welded location of the proximal end side coil body 20 and the core shaft 10 can be arranged in an extremely well-balanced manner along the circumferential direction over the entire specific range Rx of the guide wire 100a, making it possible to improve the torquability extremely effectively. In the guide wire 100a of the second embodiment, the means of joining between the proximal end side coil body 20 and the core shaft 10 at the position of the proximal end of the core shaft 10 and the intermediate position Z1 is brazing, and thus, melting of the base material can be suppressed by adopting joining by brazing at the position of the proximal end of the core shaft 10 and the intermediate position Z1, and high-strength joint can be achieved by using joining by welding at each axial specific position Pa.

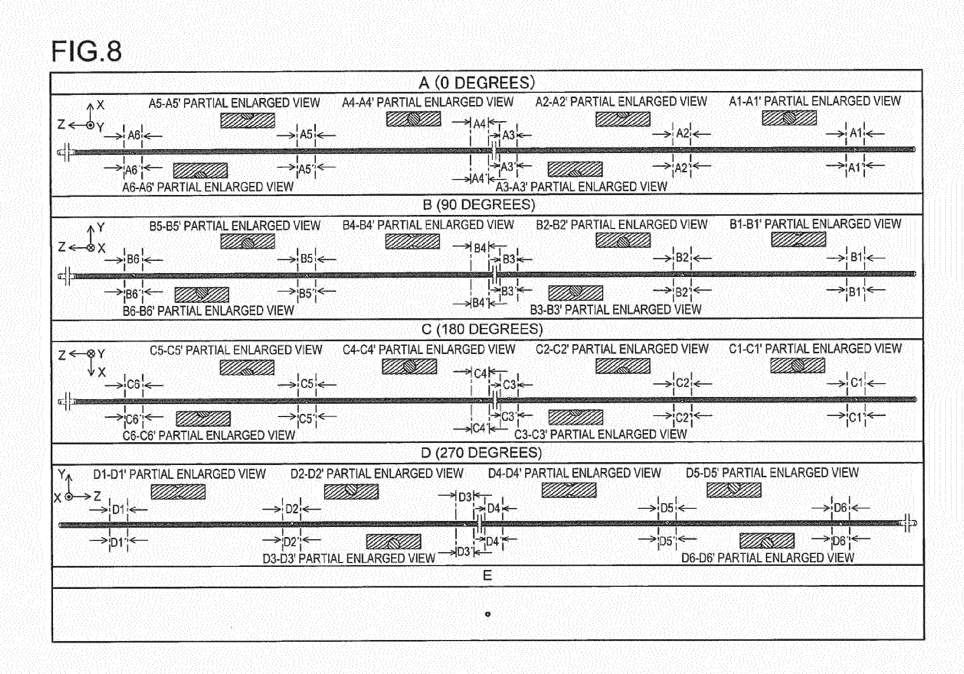

FIG. 8 is an explanatory diagram illustrating in detail the shape of the guide wire 100a of the second embodiment. Column A in FIG. 8 illustrates a top view of the guide wire 100a (as viewed from the 0° direction), column B in FIG. 8 illustrates a front view of the guide wire 100a (as viewed from the 90° direction), column C in FIG. 8 illustrates a bottom view of the guide wire 100a (as viewed from the 180° direction), column D in FIG. 8 illustrates a rear view of the guide wire 100a (as viewed from the 270° direction), and column E in FIG. 8 illustrates a left side view of the guide wire 100a (as viewed from the Z-axis positive direction). A right side view of the guide wire 100a (as viewed from the Z-axis negative direction) is the same as the left side view of the guide wire 100a illustrated in column E in FIG. 8, and is therefore omitted.

In columns A to C in FIG. 8, of the two omitted parts, the dimension on the drawing of the omitted part on the right side is 125.8 cm, the dimension on the drawing of the omitted part on the left side is 133.1 cm, and the dimension on the drawing in the entire length direction (Z-axis direction) of the guide wire 100a is 282.6 cm. In column D in FIG. 8, of the two omitted parts, the dimension on the drawing of the omitted part on the left side is 125.8 cm, the dimension on the drawing of the omitted part on the right side is 133.1 cm, and the dimension on the drawing in the entire length direction (Z-axis direction) of the guide wire 100a is 282.6 cm. In columns A to E in FIG. 8, the portion represented by the solid line is the proximal end side coil body 20 of the guide wire 100a, and is a portion for which design registration is to be received as a partial design. In columns A to D in FIG. 8, a partially enlarged view of each part is also illustrated.

### C. Third Embodiment:

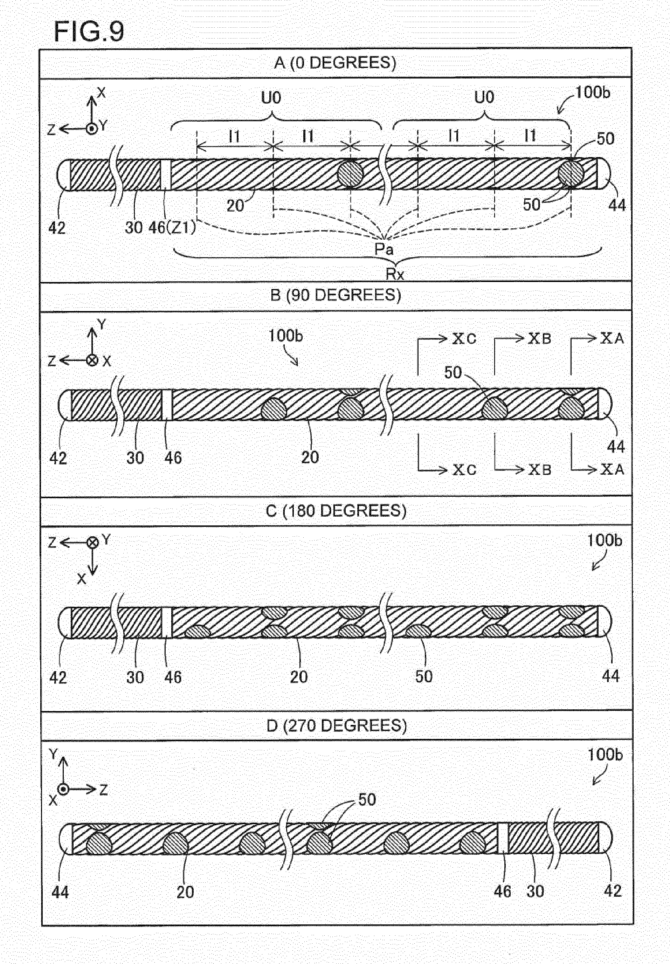

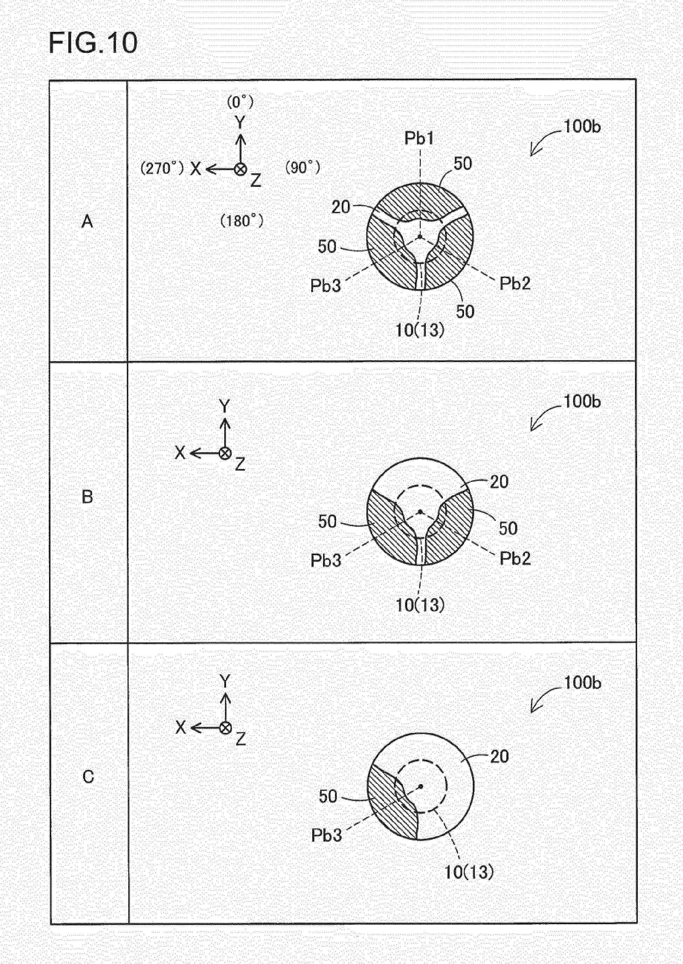

FIGS. 9 and 10 are explanatory diagrams illustrating the arrangement of the welded portions 50 in a guide wire 100b of a third embodiment. In the following, a configuration the same as that of the guide wire 100 of the first embodiment described above out of the configuration of the guide wire 100b of the third embodiment will be referred to by the same reference numerals, and a description thereof will be omitted where appropriate. Column A in FIG. 9 schematically illustrates an external configuration of the guide wire 100b as viewed from above (0° direction), column B in FIG. 9 schematically illustrates an external configuration of the guide wire 100b as viewed from the near side (90° direction), column C in FIG. 9 schematically illustrates an external configuration of the guide wire 100b as viewed from below (180° direction), and column D in FIG. 9 schematically illustrates an external configuration of the guide wire 100b as viewed from the far side (270° direction). Column A in FIG. 10 schematically illustrates a configuration of a transverse cross section (XY cross section) of the guide wire 100b at a position XA-XA in column B in FIG. 9, column B in FIG. 10 schematically illustrates a configuration of a transverse cross section (XY cross section) of the guide wire 100b at a position XB-XB in column B in FIG. 9, and column C in FIG. 10 schematically illustrates a configuration of a transverse cross section (XY cross section) of the guide wire 100b at a position XC-XC in column B in FIG. 9. In columns A to C in FIG. 10, the details of the configuration of the guide wire 100b other than the welded portion 50 are omitted.

The guide wire 100b of the third embodiment is different from the guide wire 100 of the first embodiment in the arrangement of the welded portions 50. That is, as illustrated in FIGS. 9 and 10, in the guide wire 100b of the third embodiment, similar to the guide wire 100 of the first embodiment, the plurality of axial specific positions Pa arranged at equal intervals along the axial direction are set within the specific range Rx. However, in the guide wire 100b of the third embodiment, the number of welded portions 50 formed at the axial specific positions Pa is different for each axial specific position Pa. More specifically, at the axial specific position Pa whose cross section is illustrated in column A in FIG. 10, three welded portions 50 are formed at the circumferential positions Pb (Pb1, Pb2, Pb3), which are the 0°, 120°, and 240° positions, respectively; at the axial specific position Pa whose cross section is illustrated in column B in FIG. 10, two welded portions 50 are formed at the circumferential positions Pb (Pb2, Pb3), which are the 120° and 240° positions, respectively; and at the axial specific position Pa whose cross section is illustrated in column C in FIG. 10, one welded portion 50 is formed at the circumferential positions Pb (Pb3), which is the 240° position.

As illustrated in FIG. 9, in the guide wire 100b of the third embodiment, a combination of three axial specific positions Pa that are continuous in the axial direction, in which the pattern of the number of welded portions 50 at each axial specific position Pa is the above-mentioned specific pattern (a pattern of three welded portions, two welded portions, and one welded portion) (hereinafter, referred to as "unit combination U0"), is repeatedly arranged along the axial direction. That is, a regular pattern is formed in which the number of welded portions 50 at each axial specific position Pa is three, two, one, three, two, one, and so on, in order starting from a position close to the proximal end of the guide wire 100b. The unit combination U0 in the present embodiment is an example of a first combination in the claims.

The method of forming the welded portions 50 in the guide wire 100b of the third embodiment is, for example, as described below. That is, a work having a configuration in which the proximal end side coil body 20 and the core shaft 10 are joined by the proximal end side joint part 44 and the intermediate joint part 46 is manufactured. The work is moved in the axial direction at a constant speed while being rotated in the circumferential direction at a constant speed, and the work is irradiated with laser light from a laser welder installed at a predetermined position at time intervals determined according to the number and positions of the welded portions 50 to be formed. If a plurality of the welded portions 50 are to be formed at a certain axial specific position Pa, the axial movement of the work may be stopped until the irradiation of the laser light at the axial specific position Pa is completed. By irradiating the work with laser light, the core shaft 10 and the proximal end side coil body 20 as the base material are heated and melted, and then solidified to form the welded portion 50. Through the above steps, the welded portion 50 is formed at each axial specific position Pa.

As described above, in the guide wire 100b of the third embodiment, similar to the guide wire 100 of the first embodiment, the proximal end side coil body 20 and the core shaft 10 are joined by welding at the axial specific position Pa being at least one position within the specific range Rx (the welded portions 50 for joining the proximal end side coil body 20 and the core shaft 10 are formed). Therefore, according to the guide wire 100b of the third embodiment, as in the guide wire 100 of the first embodiment, the torquability can be improved while avoiding an excessive increase in the rigidity of the proximal end side portion of the guide wire 100b.

In the guide wire 100b of the third embodiment, similar to the guide wire 100 of the first embodiment, the plurality of axial specific positions Pa exist within the specific range Rx, and thus, the torquability can be effectively improved due to the presence of the plurality of welded portions 50. In the guide wire 100b of the third embodiment, similar to the guide wire 100 of the first embodiment, the plurality of axial specific positions Pa are arranged at equal intervals along the axial direction of the core shaft 10, and thus, the welded locations of the proximal end side coil body 20 and the core shaft 10 can be arranged in a well-balanced manner along the axial direction, so that the torquability can be further effectively improved.

In the guide wire 100b of the third embodiment, the number of welded portions 50 at one axial specific position Pa is different from the number of welded portions 50 at another axial specific position Pa. Therefore, according to the guide wire 100b of the third embodiment, a technician can recognize the rotation angle of the guide wire 100b from the appearance of the guide wire 100b, and thus, the operability and the convenience can be improved.

In the guide wire 100b of the third embodiment, the unit combination U0 is a combination of the plurality of axial specific positions Pa continuous along the axial direction, and a plurality of the unit combinations U0 having the pattern of the number of welded portions 50 at each axial specific position Pa being a specific pattern are arranged along the axial direction. Therefore, according to the guide wire 100b of the third embodiment, the regular appearance of the guide wire 100b can be generated over the entire specific range Rx, a technician can easily recognize the rotation angle of the guide wire 100b according to the appearance of the guide wire 100b, and thus, the operability and the convenience can be effectively improved.

As will be described below, the guide wire 100b of the third embodiment has the configuration similar to the guide wire 100 of the first and second embodiments, and therefore exhibits the effect similar to that of the guide wire 100 of the first and second embodiments. That is, in the guide wire 100b of the third embodiment, the proximal end side coil body 20 is joined to the core shaft 10 by welding at a plurality of positions different from each other along the circumferential direction at at least one axial specific position Pa, and thus, the torquability can be effectively improved due to the presence of the plurality of welded portions 50. In the guide wire 100b of the third embodiment, the means of joining between the proximal end side coil body 20 and the core shaft 10 at the position of the proximal end of the core shaft 10 and the intermediate position Z1 is brazing, and thus, at the position of the proximal end of the core shaft 10 and the intermediate position Z1, melting of the base material can be suppressed by adopting joining by brazing, and high-strength joint can be achieved by using joining by welding at each axial specific position Pa.

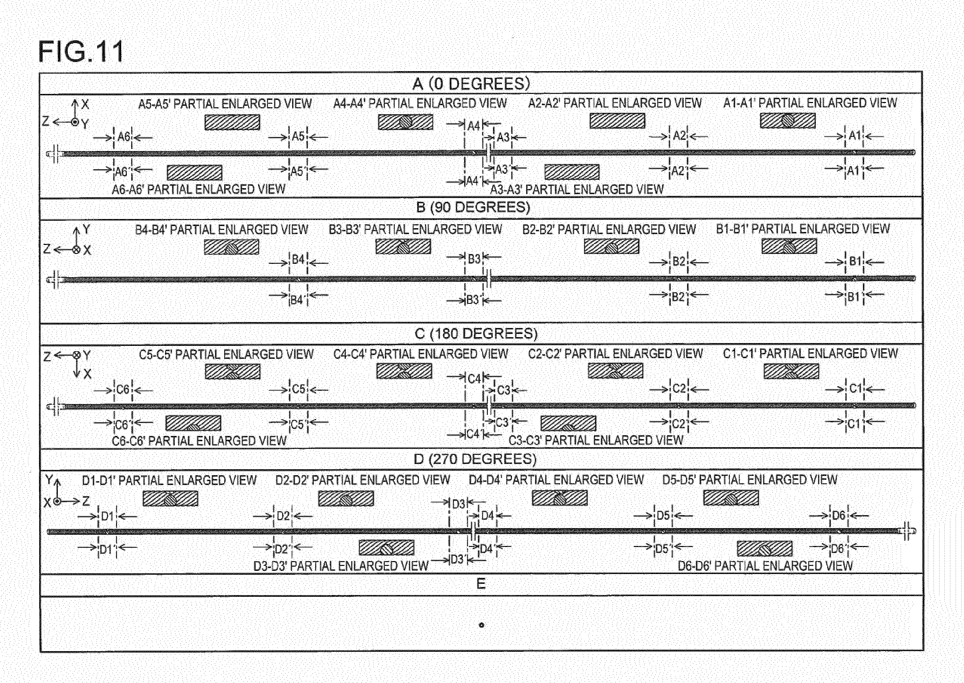

FIG. 11 is an explanatory diagram illustrating in detail the shape of the guide wire 100b of the third embodiment. Column A in FIG. 11 illustrates a top view of the guide wire 100b (as viewed from the 0° direction), column B in FIG. 11 illustrates a front view of the guide wire 100b (as viewed from the 90° direction), column C in FIG. 11 illustrates a bottom view of the guide wire 100b (as viewed from the 180° direction), column D in FIG. 11 illustrates a rear view of the guide wire 100b (as viewed from the 270° direction), and column E in FIG. 11 illustrates a left side view of the guide wire 100b (as viewed from the Z-axis positive direction). A right side view of the guide wire 100b (as viewed from the Z-axis negative direction) is the same as the left side view of the guide wire 100b illustrated in column E in FIG. 11, and is therefore omitted.

In columns A to C in FIG. 11, of the two omitted parts, the dimension on the drawing of the omitted part on the right side is 125.8 cm, the dimension on the drawing of the omitted part on the left side is 133.1 cm, and the dimension on the drawing in the entire length direction (Z-axis direction) of the guide wire 100b is 282.6 cm. In column D in FIG. 11, of the two omitted parts, the dimension on the drawing of the omitted part on the left side is 125.8 cm, the dimension on the drawing of the omitted part on the right side is 133.1 cm, and the dimension on the drawing in the entire length direction (Z-axis direction) of the guide wire 100b is 282.6 cm. In columns A to E in FIG. 11, the portion represented by the solid line is the proximal end side coil body 20 of the guide wire 100b, and is a portion for which design registration is to be received as a partial design. In columns A to D in FIG. 11, a partially enlarged view of each part is also illustrated.

### D. Fourth Embodiment:

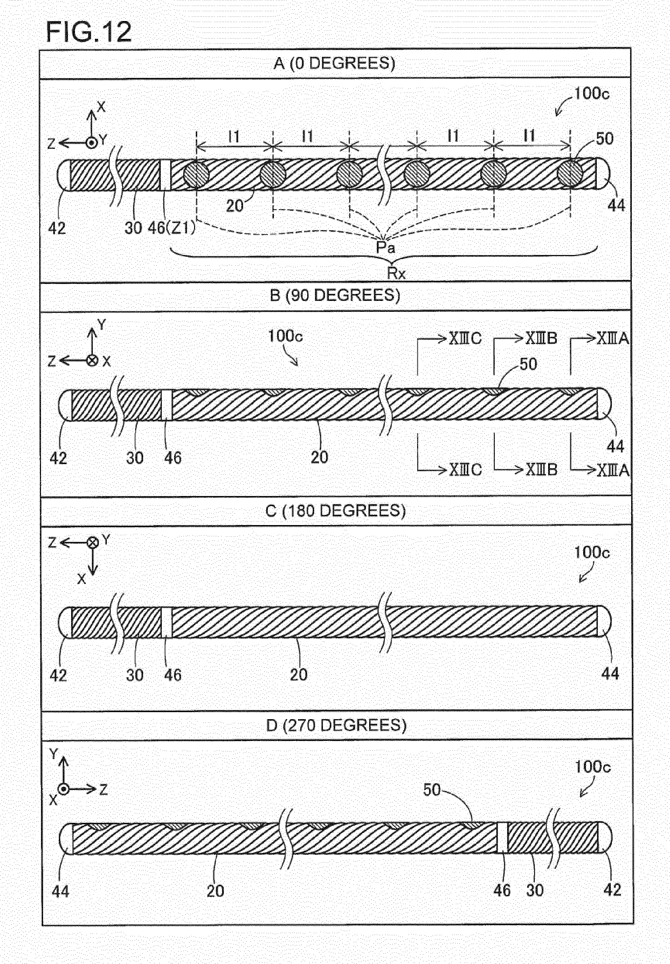

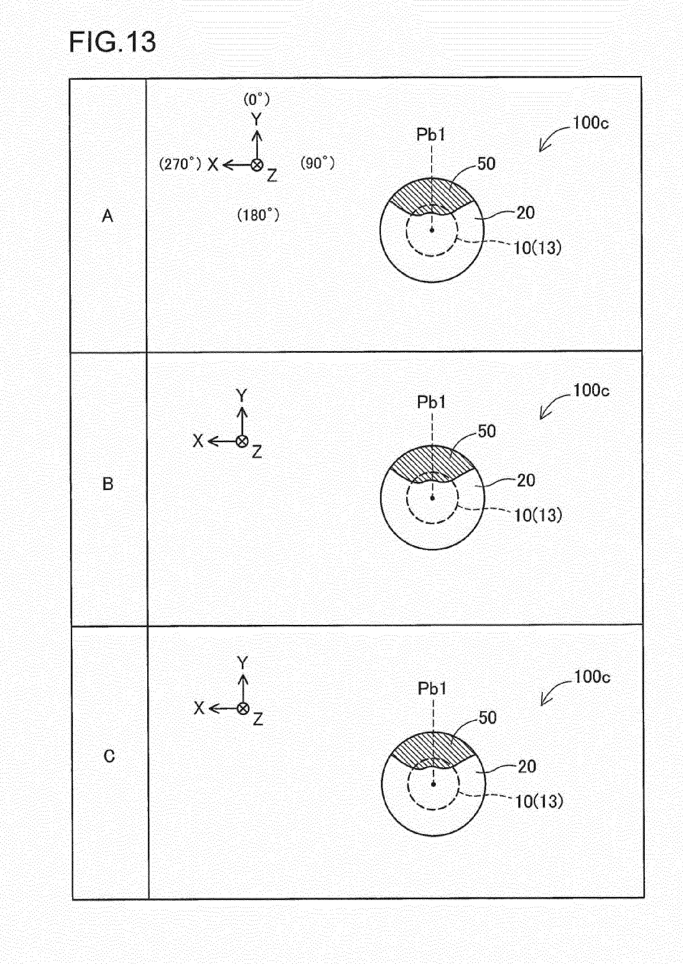

FIGS. 12 and 13 are explanatory diagrams illustrating the arrangement of the welded portions 50 in a guide wire 100c of a fourth embodiment. In the following, a configuration the same as that of the guide wire 100 of the first embodiment described above out of the configuration of the guide wire 100c of the fourth embodiment will be referred to by the same reference numerals, and a description thereof will be omitted where appropriate. Column A in FIG. 12 schematically illustrates an external configuration of the guide wire 100c as viewed from above (0° direction), column B in FIG. 12 schematically illustrates an external configuration of the guide wire 100c as viewed from the near side (90° direction), column C in FIG. 12 schematically illustrates an external configuration of the guide wire 100c as viewed from below (180° direction), and column D in FIG. 12 schematically illustrates an external configuration of the guide wire 100c as viewed from the far side (270° direction). Column A in FIG. 13 schematically illustrates a configuration of a transverse cross section (XY cross section) of the guide wire 100c at a position XIIIA-XIIIA in column B in FIG. 12, column B in FIG. 13 schematically illustrates a configuration of a transverse cross section (XY cross section) of the guide wire 100c at a position XIIIB-XIIIB in column B in FIG. 12, and column C in FIG. 13 schematically illustrates a configuration of a transverse cross section (XY cross section) of the guide wire 100c at a position XIIIC-XIIIC in column B in FIG. 12. In columns A to C in FIG. 13, the details of the configuration of the guide wire 100c other than the welded portion 50 are omitted.

The guide wire 100c of the fourth embodiment is different from the guide wire 100 of the first embodiment in the arrangement of the welded portions 50. That is, as illustrated in FIG. 12 and FIG. 13, in the guide wire 100c of the fourth embodiment, similar to the guide wire 100 of the first embodiment, the plurality of axial specific positions Pa arranged at equal intervals along the axial direction are set within the specific range Rx, and one welded portion 50 is formed at each axial specific position Pa. However, in the guide wire 100c of the fourth embodiment, the circumferential positions Pb (Pb1) of the welded portions 50 at the axial specific positions Pa are the same as each other (all are at the 0° position). That is, in the guide wire 100c of the fourth embodiment, the proximal end side coil body 20 is joined to the core shaft 10 by welding at the same position along the circumferential direction at each axial specific position Pa.

The method of forming the welded portions 50 in the guide wire 100c of the fourth embodiment is, for example, as described below. That is, a work having a configuration in which the proximal end side coil body 20 and the core shaft 10 are joined by the proximal end side joint part 44 and the intermediate joint part 46 is manufactured. The work is moved in the axial direction at a constant speed in a state of not being rotated in the circumferential direction, and the work is irradiated with laser light from a laser welder installed at a predetermined position at regular time intervals. By irradiating the work with laser light, the core shaft 10 and the proximal end side coil body 20 as the base material are heated and melted, and then solidified to form the welded portion 50. Through the above steps, the welded portion 50 is formed at each axial specific position Pa.

As described above, in the guide wire 100c of the fourth embodiment, similar to the guide wire 100 of the first embodiment, the proximal end side coil body 20 and the core shaft 10 are joined by welding at the axial specific position Pa being at least one position within the specific range Rx (the welded portions 50 for joining the proximal end side coil body 20 and the core shaft 10 are formed). Therefore, according to the guide wire 100c of the fourth embodiment, as in the guide wire 100 of the first embodiment, the torquability can be improved while avoiding an excessive increase in the rigidity of the proximal end side portion of the guide wire 100c.

In the guide wire 100c of the fourth embodiment, similar to the guide wire 100 of the first embodiment, the plurality of axial specific positions Pa exist within the specific range Rx, and thus, the torquability can be effectively improved due to the presence of the plurality of welded portions 50. In the guide wire 100c of the fourth embodiment, similar to the guide wire 100 of the first embodiment, the plurality of axial specific positions Pa are arranged at equal intervals along the axial direction of the core shaft 10, and thus, the welded locations of the proximal end side coil body 20 and the core shaft 10 can be arranged in a well-balanced manner along the axial direction, so that the torquability can be further effectively improved.

In the guide wire 100c of the fourth embodiment, the circumferential positions Pb of the welded portions 50 at the axial specific positions Pa are the same position as each other. Therefore, according to the guide wire 100c of the fourth embodiment, every time the guide wire 100c is rotated by 360° in the circumferential direction, intentional bouncing can be generated, and thus, a technician can easily perform a specific operation of the guide wire 100c (for example, escape from a false cavity or guide to a branch pipe), so that the operability and the convenience can be improved. Moreover, it is not necessary to stop the axial movement of the work at the time of laser welding for forming the welded portions 50 at each axial specific position Pa, and thus, the time required for forming the welded portions 50 can be shortened, making it possible to improve the efficiency of manufacturing the guide wire 100c.

As will be described below, the guide wire 100c of the fourth embodiment has the configuration similar to that of the guide wire 100 of the first through third embodiments, and therefore exhibits the effect similar to that of the guide wire 100 of the first through third embodiments. In the guide wire 100c of the fourth embodiment, the means of joining between the proximal end side coil body 20 and the core shaft 10 at the position of the proximal end of the core shaft 10 and the intermediate position Z1 is brazing, and thus, at the position of the proximal end of the core shaft 10 and the intermediate position Z1, melting of the base material can be suppressed by adopting joining by brazing, so that high-strength joint can be achieved by using joining by welding at each axial specific position Pa.

### E. Fifth Embodiment:

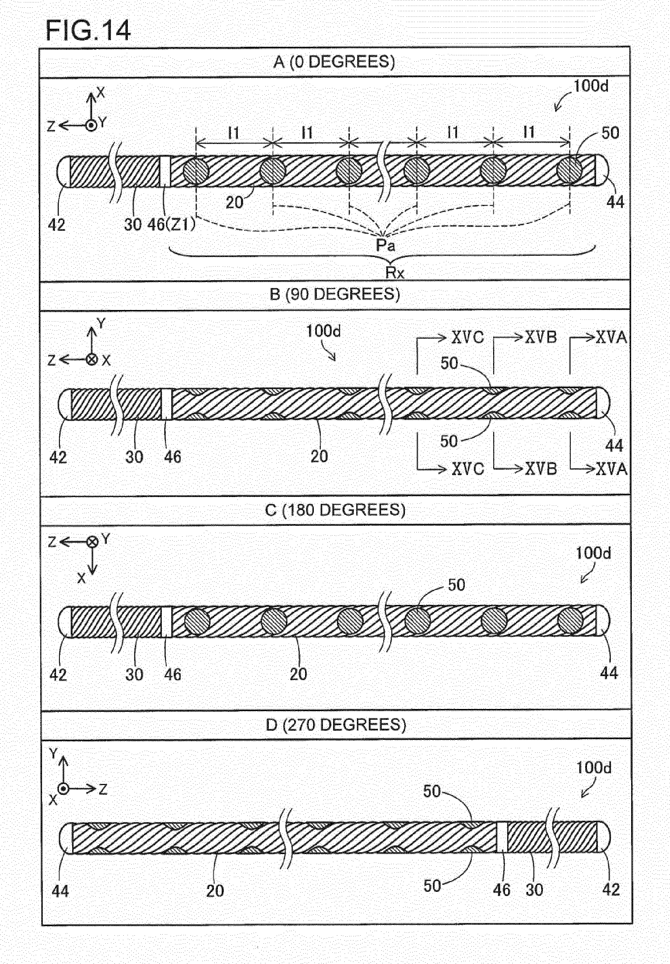

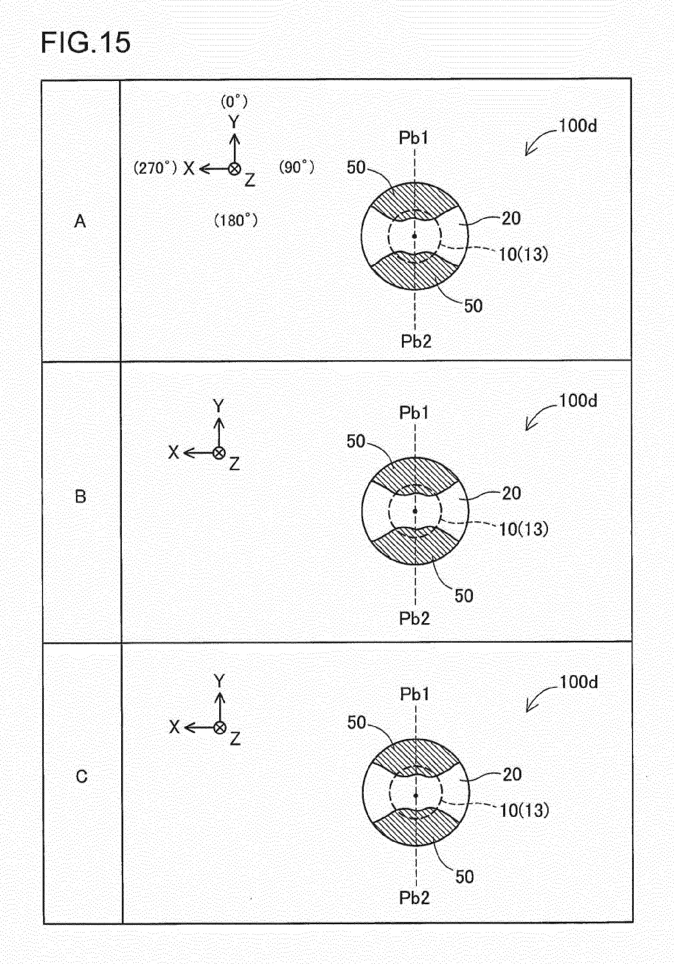

FIGS. 14 and 15 are explanatory diagrams illustrating the arrangement of the welded portions 50 in a guide wire 100d of a fifth embodiment. In the following, a configuration the same as that of the guide wire 100 of the first embodiment described above out of the configuration of the guide wire 100d of the fifth embodiment will be referred to by the same reference numerals, and a description thereof will be omitted where appropriate. Column A in FIG. 14 schematically illustrates an external configuration of the guide wire 100d as viewed from above (0° direction), column B in FIG. 14 schematically illustrates an external configuration of the guide wire 100d as viewed from the near side (90° direction), column C in FIG. 14 schematically illustrates an external configuration of the guide wire 100d as viewed from below (180° direction), and column D in FIG. 14 schematically illustrates an external configuration of the guide wire 100d as viewed from the far side (270° direction). Column A in FIG. 15 schematically illustrates a configuration of a transverse cross section (XY cross section) of the guide wire 100d at a position XVA-XVA in column B in FIG. 14, column B in FIG. 15 schematically illustrates a configuration of a transverse cross section (XY cross section) of the guide wire 100d at a position XVB-XVB in column B in FIG. 14, and column C in FIG. 15 schematically illustrates a configuration of a transverse cross section (XY cross section) of the guide wire 100d at a position XVC-XVC in column B in FIG. 14. In columns A to C in FIG. 15, the details of the configuration of the guide wire 100d other than the welded portion 50 are omitted.

The guide wire 100d of the fifth embodiment is different from the guide wire 100 of the first embodiment in the arrangement of the welded portions 50. That is, as illustrated in FIGS. 14 and 15, in the guide wire 100d of the fifth embodiment, similar to the guide wire 100 of the first embodiment, the plurality of axial specific positions Pa arranged at equal intervals along the axial direction are set within the specific range Rx. However, in the guide wire 100d of the fifth embodiment, two welded portions 50 are formed at each axial specific position Pa. In the guide wire 100d of the fifth embodiment, the combinations of the circumferential positions Pb (Pb1, Pb2) of two welded portions 50 at the axial specific positions Pa are the same as each other (all are combinations of 0° and 180° positions). In the guide wire 100d of the fifth embodiment, the circumferential positions Pb (Pb1, Pb2) of the welded portions 50 at each axial specific position Pa are arranged at equal intervals (180° intervals) along the circumferential direction.

The method of forming the welded portions 50 in the guide wire 100d of the fifth embodiment is, for example, as described below. That is, a work having a configuration in which the proximal end side coil body 20 and the core shaft 10 are joined by the proximal end side joint part 44 and the intermediate joint part 46 is manufactured. The work is moved in the axial direction at a constant speed with rotation in the circumferential direction being restricted, and the work is irradiated with laser light from a laser welder installed at a predetermined position at regular time intervals. By irradiating the work with laser light, the core shaft 10 and the proximal end side coil body 20 as the base material are heated and melted, and then solidified to form the welded portion 50. Through the above steps, one welded portion 50 is formed at each axial specific position Pa. After that, the work is rotated by 180° in the circumferential direction, after which the rotation in the circumferential direction is restricted, the work is moved in the opposite axial direction at a constant speed, and the work is irradiated with laser light from a laser welder installed at a predetermined position at regular time intervals. By irradiating the work with laser light, the core shaft 10 and the proximal end side coil body 20 as the base material are heated and melted, and then solidified to form the welded portion 50. Through the above steps, the other welded portion 50 at each axial specific position Pa is formed, and as a result, two welded portions 50 are formed at each axial specific position Pa. Instead of the above method, the welded portions 50 in the guide wire 100d of the fifth embodiment may be formed by forming two welded portions 50 at one axial specific position Pa by moving the work by a predetermined distance in the axial direction, performing a first irradiation of the laser light, rotating the work by 180°, and performing a second irradiation of the laser beam in that order, and repeatedly executing this process at each axial specific position Pa.

As described above, in the guide wire 100d of the fifth embodiment, similar to the guide wire 100 of the first embodiment, the proximal end side coil body 20 and the core shaft 10 are joined by welding at an axial specific position Pa being at least one position within the specific range Rx (the welded portions 50 for joining the proximal end side coil body 20 and the core shaft 10 are formed). Therefore, according to the guide wire 100d of the fifth embodiment, as in the guide wire 100 of the first embodiment, the torquability can be improved while avoiding an excessive increase in the rigidity of the proximal end side portion of the guide wire 100d.

In the guide wire 100d of the fifth embodiment, similar to the guide wire 100 of the first embodiment, the plurality of axial specific positions Pa exist within the specific range Rx, and thus, the torquability can be effectively improved due to the presence of the plurality of welded portions 50. In the guide wire 100d of the fifth embodiment, similar to the guide wire 100 of the first embodiment, the plurality of axial specific positions Pa are arranged at equal intervals along the axial direction of the core shaft 10, and thus, the welded locations of the proximal end side coil body 20 and the core shaft 10 can be arranged in a well-balanced manner along the axial direction, so that the torquability can be further effectively improved.

As will be described below, the guide wire 100d of the fifth embodiment has the configuration similar to that of the guide wire 100 of the first through fourth embodiments, and therefore exhibits the effect similar to that of the guide wire 100 of the first through fourth embodiments. That is, in the guide wire 100d of the fifth embodiment, the proximal end side coil body 20 is joined to the core shaft 10 by welding at a plurality of positions different from each other along the circumferential direction at each axial specific position Pa, and thus, the torquability can be effectively improved due to the presence of the plurality of welded portions 50 at each axial specific position Pa. In the guide wire 100d of the fifth embodiment, the circumferential positions Pb of the welded portions 50 at each axial specific position Pa are arranged at equal intervals along the circumferential direction, and thus, the welded locations of the proximal end side coil body 20 and the core shaft 10 can be arranged in a well-balanced manner along the circumferential direction at each axial specific position Pa, so that the torquability can be further effectively improved. In the guide wire 100d of the fifth embodiment, the means of joining between the proximal end side coil body 20 and the core shaft 10 at the position of the proximal end of the core shaft 10 and the intermediate position Z1 is brazing, and thus, at the position of the proximal end of the core shaft 10 and the intermediate position Z1, melting of the base material can be suppressed by adopting joining by brazing, and high-strength joint can be achieved by using joining by welding at each axial specific position Pa.

### F. Sixth Embodiment:

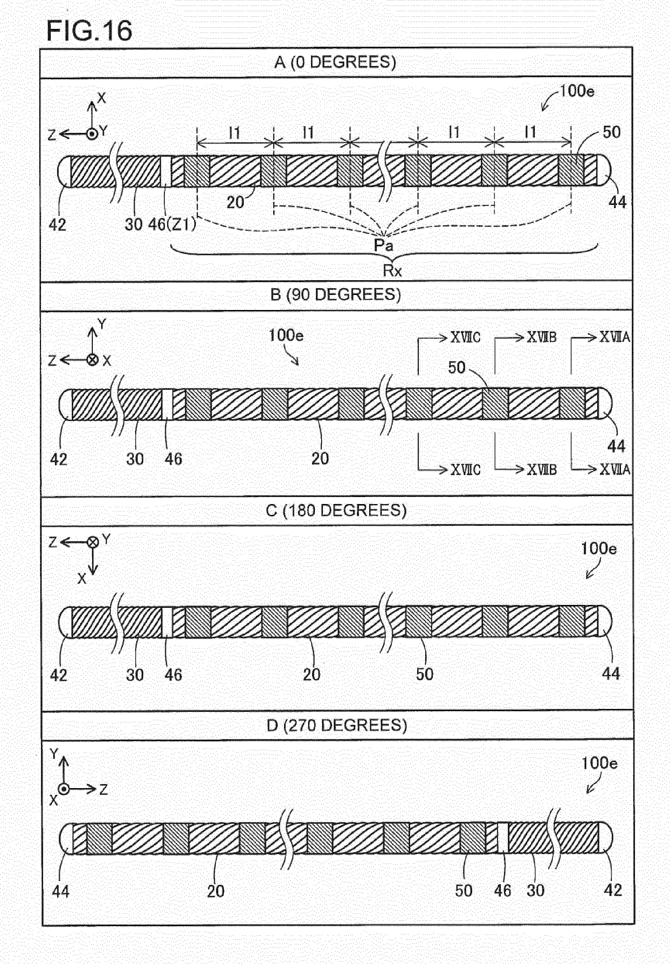

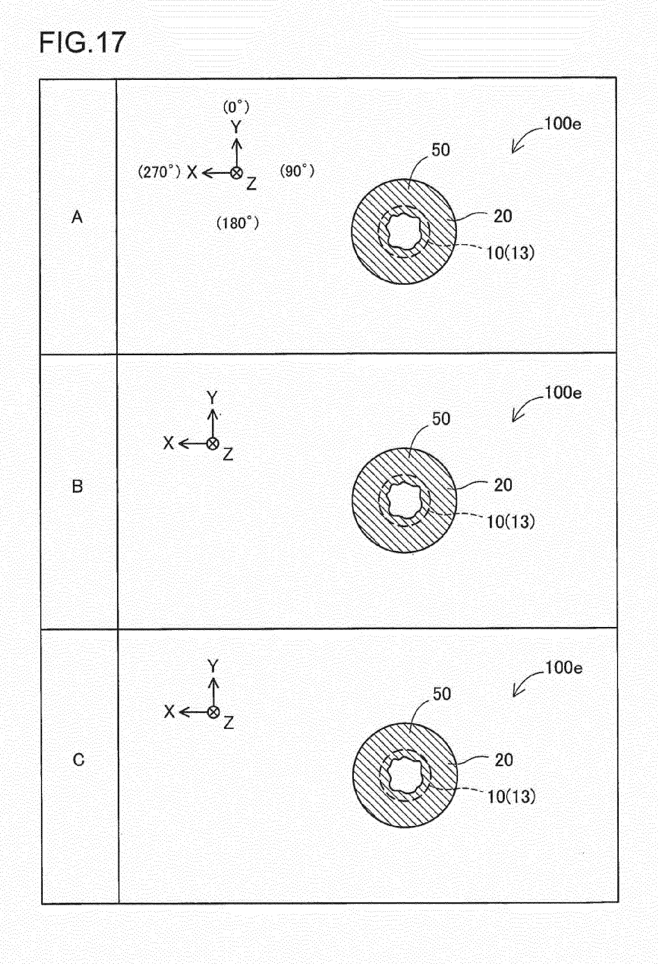

FIGS. 16 and 17 are explanatory diagrams illustrating the arrangement of the welded portions 50 in a guide wire 100e of a sixth embodiment. In the following, a configuration the same as that of the guide wire 100 of the first embodiment described above out of the configuration of the guide wire 100e of the sixth embodiment will be referred to by the same reference numerals, and a description thereof will be omitted where appropriate. Column A in FIG. 16 schematically illustrates an external configuration of the guide wire 100e as viewed from above (0° direction), column B in FIG. 16 schematically illustrates an external configuration of the guide wire 100e as viewed from the near side (90° direction), column C in FIG. 16 schematically illustrates an external configuration of the guide wire 100e as viewed from below (180° direction), and column D in FIG. 16 schematically illustrates an external configuration of the guide wire 100e as viewed from the far side (270° direction). Column A in FIG. 17 schematically illustrates a configuration of a transverse cross section (XY cross section) of the guide wire 100e at a position XVIIA-XVIIA in column B in FIG. 16, column B in FIG. 17 schematically illustrates a configuration of a transverse cross section (XY cross section) of the guide wire 100e at a position XVIIB-XVIIB in column B in FIG. 16, and column C in FIG. 17 schematically illustrates a configuration of a transverse cross section (XY cross section) of the guide wire 100e at a position XVIIC-XVIIC in column B in FIG. 16. In columns A to C in FIG. 17, details of the configuration of the guide wire 100e other than the welded portion 50 are omitted.

The guide wire 100e of the sixth embodiment is different from the guide wire 100 of the first embodiment in the arrangement of the welded portions 50. That is, as illustrated in FIG. 16 and FIG. 17, in the guide wire 100e of the sixth embodiment, similar to the guide wire 100 of the first embodiment, the plurality of axial specific positions Pa arranged at equal intervals along the axial direction are set within the specific range Rx, and one welded portion 50 is formed at each axial specific position Pa. However, in the guide wire 100e of the sixth embodiment, the proximal end side coil body 20 is joined to the core shaft 10 by welding all the way along the circumferential direction at each axial specific position Pa. That is, each welded portion 50 is formed around the entire outer periphery of the guide wire 100e in the XY cross section.

The method of forming each welded portion 50 in the guide wire 100e of the sixth embodiment is, for example, as described below. That is, a work having a configuration in which the proximal end side coil body 20 and the core shaft 10 are joined by the proximal end side joint part 44 and the intermediate joint part 46 is manufactured. The work is moved by a predetermined distance in the axial direction, and the work is rotated by 360° in the circumferential direction while irradiating the work with laser light from a laser welder installed at a predetermined position. After that, the work is moved by a predetermined distance in the axial direction, and the work is rotated by 360° in the circumferential direction while irradiating the work with laser light from a laser welder installed at a predetermined position. By repeatedly executing such a process for the number of locations of the axial specific positions Pa, the welded portion 50 is formed at each axial specific position Pa.

As described above, in the guide wire 100e of the sixth embodiment, similar to the guide wire 100 of the first embodiment, the proximal end side coil body 20 and the core shaft 10 are joined by welding at the axial specific position Pa being at least one position within the specific range Rx (the welded portions 50 for joining the proximal end side coil body 20 and the core shaft 10 are formed). Therefore, according to the guide wire 100e of the sixth embodiment, as in the guide wire 100 of the first embodiment, the torquability can be improved while avoiding an excessive increase in the rigidity of the proximal end side portion of the guide wire 100e.

In the guide wire 100e of the sixth embodiment, similar to the guide wire 100 of the first embodiment, the plurality of axial specific positions Pa exist within the specific range Rx, and thus, the torquability can be effectively improved due to the presence of the plurality of welded portions 50. In the guide wire 100e of the sixth embodiment, similar to the guide wire 100 of the first embodiment, the plurality of axial specific positions Pa are arranged at equal intervals along the axial direction of the core shaft 10, and thus, the welded locations of the proximal end side coil body 20 and the core shaft 10 can be arranged in a well-balanced manner along the axial direction, so that the torquability can be further effectively improved.

In the guide wire 100e of the sixth embodiment, the proximal end side coil body 20 is joined to the core shaft 10 by welding all the way along the circumferential direction at each axial specific position Pa. Therefore, according to the guide wire 100e of the sixth embodiment, the welding strength per location can be increased, and the torquability can be effectively improved. Moreover, a technician can easily recognize the insertion length of the guide wire 100e by the appearance and/or tactile sensation of the guide wire 100e, and thus, the operability and the convenience can be improved.

As will be described below, the guide wire 100e of the sixth embodiment has the configuration similar to that the guide wire 100 of the first through fifth embodiments, and therefore exhibits the effect similar to that of the guide wire 100 of the first through fifth embodiments. That is, in the guide wire 100e of the sixth embodiment, the means of joining between the proximal end side coil body 20 and the core shaft 10 at the position of the proximal end of the core shaft 10 and the intermediate position Z1 is brazing, and thus, at the position of the proximal end of the core shaft 10 and the intermediate position Z1, melting of the base material can be suppressed by adopting joining by brazing, and high-strength joint can be achieved by using joining by welding at each axial specific position Pa.

### G. Modifications:

The techniques disclosed herein are not limited to the above-described embodiments, and may be modified into various modes without departing from the spirit of the above-described embodiments. For example, the following modifications can be applied.

The configuration of the guide wire in each of the above embodiments is merely an example, and various modifications are possible. For example, in each of the above embodiments, the plurality of axial specific positions Pa are arranged at equal intervals along the axial direction; however, the plurality of axial specific positions Pa may be arranged at non-equal intervals along the axial direction. For example, the distance between two axial specific positions Pa adjacent to each other in the axial direction may be configured to gradually increase in order from the proximal end side to 20 mm, 30 mm, 40 mm, and so on, or conversely, may be configured to gradually decrease in order from the proximal end side to 300 mm, 290 mm, 280 mm, and so on. Alternatively, the distance between two axial specific positions Pa adjacent to each other in the axial direction may be configured such that a specific pattern is repeated in order from the proximal end side, such as 20 mm, 30 mm, 40 mm, 20 mm, 30 mm, 40 mm, and so on.

In each of the above embodiments, the plurality of axial specific positions Pa (that is, positions where the welded portions 50 are formed) are set along the guide wire 100, but only one axial specific position Pa may be set along the guide wire 100.

In each of the above embodiments, the proximal end side coil body 20 and the distal end side coil body 30 may be configured as an integral coil body. In this case, for example, the welded portions 50 may be formed in a part of the coil body on the proximal end side (a portion corresponding to the proximal end side coil body 20).

In each of the above embodiments, the welded portions 50 for joining the proximal end side coil body 20 and the core shaft 10 are formed by welding; however, in addition to this, only the proximal end side coil body 20 may be melted by irradiation with laser light to form melted portions for marking so that a technician can easily know the rotation angle and the insertion length.

In each of the above embodiments, the proximal end side coil body 20 and the distal end side coil body 30 are configured such that the wire is tightly wound, but the proximal end side coil body 20 and/or the distal end side coil body 30 may have a configuration in which a wire is roughly wound. In each of the above embodiments, the proximal end side coil body 20 and the distal end side coil body 30 are configured to have a hollow cylindrical shape by spirally winding one wire; however, the proximal end side coil body 20 and/or the distal end side coil body 30 may have a configuration formed into a hollow cylindrical shape by spirally winding a plurality of wires, or may have a configuration formed into a hollow cylindrical shape by spirally winding a single strand formed by twisting together a plurality of wires, or may have a configuration formed into a hollow cylindrical shape by spirally winding a plurality strands each formed by twisting together a plurality of wires.

In each of the above embodiments, the core shaft 10 includes the small-diameter portion 11, the tapered portion 12, and the large-diameter portion 13; however, the core shaft 10 may not have at least one of these three portions, or may have other portions in addition to these three portions.

The material of each member in each of above-described embodiments is merely an example, and various modifications are possible. The method for manufacturing the guide wire in each of the above embodiments is merely an example, and various modifications are possible. For example, in each of the above embodiments, the proximal end side coil body 20 and the core shaft 10 are joined at the proximal end side joint part 44 and the intermediate joint part 46, and then the proximal end side coil body 20 and the core shaft 10 are joined by welding; however, the proximal end side joint part 44 and the intermediate joint part 46 may be formed after the proximal end side coil body 20 and the core shaft 10 have been joined by welding. Alternatively, the proximal end side coil body 20 and the core shaft 10 may be joined by welding while winding the proximal end side coil body 20 around the core shaft 10.

### DESCRIPTION OF REFERENCE NUMERALS

10 Core shaft
11 Small-diameter portion
12 Tapered portion
13 Large-diameter portion
20 Proximal end side coil body
30 Distal end side coil body
42 Distal end side joint part
44 Proximal end side joint part
46 Intermediate joint part
50 Welded portion
100 Guide wire
Pa Axial specific position
Pb Circumferential position
Rx Specific range
U0 Unit combination
Z1 Intermediate position

## Claims

1. A guide wire comprising:
a core shaft; and
a coil body wound around at least a portion on a proximal end side of the core shaft; wherein
the coil body is joined with the core shaft by a predetermined joint means at a position of a proximal end of the core shaft, and a first position along an axial direction of the guide wire between the proximal end and a distal end of the core shaft, and
the coil body is joined with the core shaft by welding at an axial specific position being at least one position within a range along the axial direction of the guide wire from the proximal end of the core shaft to the first position.

2. The guide wire according to claim 1, wherein
the guide wire includes a plurality of the axial specific positions.

3. The guide wire according to claim 2, wherein
the plurality of axial specific positions are arranged at equal intervals along the axial direction of the guide wire.

4. The guide wire according to claim 2 or 3, wherein
the coil body is joined with the core shaft by welding at a part along a circumferential direction of the guide wire at at least one of the plurality of axial specific positions.

5. The guide wire according to claim 4, wherein
the coil body is joined with the core shaft by welding at a plurality of locations different from each other along the circumferential direction of the guide wire at at least one of the plurality of axial specific positions.

6. The guide wire according to claim 5, wherein
the plurality of locations different from each other along the circumferential direction of the guide wire are arranged at equal intervals along the circumferential direction of the guide wire.

7. The guide wire according to claim 5 or 6, wherein
the number of welded locations at one of the plurality of axial specific positions is different from the number of welded locations at another of the plurality of axial specific positions.

8. The guide wire according to claim 7, wherein
a plurality of first combinations are arranged along the axial direction of the guide wire, the first combination being composed of a plurality of axial specific positions continuous along the axial direction of the guide wire in which a pattern of the number of welded locations at each of the plurality of axial specific positions is a specific pattern.

9. The guide wire according to any one of claims 4 to 8, wherein
a position of a welded location along a circumferential direction of the guide wire at one of the plurality of axial specific positions differs from a position of a welded location along the circumferential direction of the guide wire at another of the plurality of axial specific positions.

10. The guide wire according to claim 9, wherein
a plurality of second combinations are arranged along the axial direction of the guide wire, the second combination being composed of a plurality of axial specific positions continuous along the axial direction of the guide wire in which a pattern of a position of a welded location along the circumferential direction of the guide wire at each of the plurality of axial specific positions is a specific pattern.

11. The guide wire according to claim 10, wherein
for the plurality of axial specific positions included in the second combination, amounts of deviation of positions of welded locations along the circumferential direction of the guide wire between any two of the plurality of axial specific positions are equal to each other, the two axial specific positions being adjacent to each other along the axial direction of the guide wire.

12. The guide wire according to any one of claims 2 to 11, wherein
the coil body is joined with the core shaft by welding over an entire circumference along the circumferential direction of the guide wire at at least one of the plurality of axial specific positions.

13. The guide wire according to claim 4, wherein
a position of a welded location along the circumferential direction of the guide wire at each of the plurality of axial specific positions is identical to each other.

14. The guide wire according to any one of claims 1 to 13, wherein
the predetermined joint means is brazing.
